(19) Europäisches Patentamt
European Patent Office
Office européen des brevets

(11) Publication number: **0 319 228 B1**

(12) **EUROPEAN PATENT SPECIFICATION**

(45) Date of publication of patent specification: **02.11.94**

(51) Int. Cl.[5]: **C07F 9/547**, C07F 9/40, A61K 31/675

(21) Application number: **88311252.6**

(22) Date of filing: **28.11.88**

(54) **Novel compounds.**

(30) Priority: **30.11.87 GB 8727988**
**16.05.88 GB 8811575**

(43) Date of publication of application:
**07.06.89 Bulletin 89/23**

(45) Publication of the grant of the patent:
**02.11.94 Bulletin 94/44**

(84) Designated Contracting States:
**AT BE CH DE ES FR GB GR IT LI LU NL SE**

(56) References cited:
**EP-A- 0 206 459**
**FR-A- 2 085 671**

(73) Proprietor: **BEECHAM GROUP plc**
**Four New Horizons Court**
**Harlequin Avenue**
**Brentford Middlesex TW8 9EP (GB)**

(72) Inventor: **Harnden, Michael Raymond**
**47 Guildford Road**
**Horsham, West Sussex, RH12 1ND (GB)**
Inventor: **Duckworth, David Malcolm**
**SmithKline Beecham plc**
**Coldharbour Road,**
**The Pinnacles, Harlow, Essex CM19 5AD (GB)**

(74) Representative: **Jones, Pauline et al**
**SmithKline Beecham plc**
**Corporate Intellectual Property**
**SB House**
**Great West Road**
**Brentford, Middlesex TW8 9BD (GB)**

EP 0 319 228 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention relates to compounds having antiviral activity, to processes for their preparation and to their use as pharmaceuticals.

EP-A-242482 (Beecham Group p.l.c.) describes a group of guanine derivatives having a 9-hydroxyalkoxy substituent, and possessing antiviral activity.

EP-A-206459 (Ceskoslovenska akademie ved) describes 9-(phosphonylmethoxyalkyl)adenines reported to exhibit biological effects (e.g. antiviral) or can be converted into compounds with such effects

A novel, structurally distinct class of compounds has now been discovered, these compounds also having antiviral activity.

Accordingly, the present invention provides a compound of formula (I), or a pharmaceutically acceptable salt thereof:

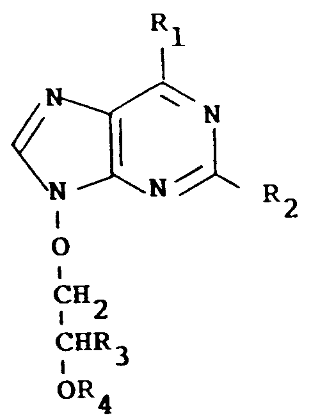

(I)

wherein

$R_1$ is hydroxy, amino, chloro or $OR_7$

wherein

$R_7$ is $C_{1-6}$ alkyl, phenyl or phenyl $C_{1-2}$ alkyl either of which phenyl moieties may be substituted by one or two substituents selected from halo, $C_{1-4}$ alkyl or $C_{1-4}$ alkoxy;

$R_2$ is amino or, when $R_1$ is hydroxy or amino, $R_2$ may also be hydrogen;

$R_3$ is hydrogen, hydroxymethyl or acyloxymethyl;

$R_4$ is a group of formula

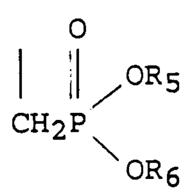

wherein

$R_5$ and $R_6$ are independently selected from hydrogen, $C_{1-6}$ alkyl and optionally substituted phenyl; or

$R_3$ and $R_4$ together are:

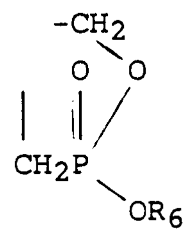

wherein

$R_6$ is as defined above.

When $R_1$ is hydroxy and $R_2$ is amino, the compound of formula (I) is a guanine derivative;
When $R_1$ is amino and $R_2$ is hydrogen, the compound of formula (I) is an adenine derivative;
When $R_1$ is hydroxy and $R_2$ is hydrogen, the compound of formula (I) is a hypoxanthine derivative; and
When $R_1$ and $R_2$ are both amino groups, the compound of formula (I) is a 2,6-diaminopurine derivative.

Often, the compound of formula (I) is a guanine or adenine derivative.

Suitable examples of the acyl group in $R_3$ when acyloxymethyl, include carboxylic acyl, such as $C_{1-7}$ alkanoyl and benzoyl optionally substituted in the phenyl ring as defined below for $R_5/R_6$. Preferred acyl groups include acetyl, propionyl, butyryl, heptanoyl and hexanoyl.

Suitable examples of $R_5$ and $R_6$ include hydrogen, methyl, ethyl, n- and iso-propyl, n-, sec-, iso- and tert-butyl, and phenyl optionally substituted by one, two or three groups or atoms selected from halogen, such as fluoro, chloro, bromo, and $C_{1-4}$ alkyl or $C_{1-4}$ alkyl or $C_{1-4}$ alkoxy wherein the alkyl moiety is selected from those listed for $R_5/R_6$ above.

Examples of $R_7$ include methyl, ethyl, n- and iso-propyl, phenyl and benzyl optionally substituted by one or two of methyl, ethyl, n- and iso-propyl, methoxy, ethoxy, n- and iso-propoxy, fluoro, chloro or bromo.

Examples of pharmaceutically acceptable salts of the compound of formula (I) are acid addition salts formed with a pharmaceutically acceptable acid such as hydrochloric acid, orthophosphoric acid and sulphuric acid. Pharmaceutically acceptable salts also include those formed with organic bases, preferably with amines, such as ethanolamines or diamines; and alkali metals, such as sodium and potassium.

As the compound of formula (I) contains a phosphonate group, suitable salts include metal salts, such as alkali metal salts, for example sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine.

It will be appreciated that some of the compounds of formula (I), especially those wherein $R_3$ is other than hydrogen, have an asymmetric centre, and therefore are capable of existing in more than one stereoisomeric form. The invention extends to each of these forms individually and to mixtures thereof, including racemates. The isomers may be separated conventionally by chromatographic methods or using a resolving agent. Alternatively, the individual isomers may be prepared by asymmetric synthesis using chiral intermediates.

The compound of formula (I) including their alkali metal salts may form solvates such as hydrates and these are included wherever a compound of formula (I) or a salt thereof is herein referred to.

It will be appreciated that, when $R_1$ is hydroxy in formula (I) the compound exists in the predominant tautomeric form of structure (IA):

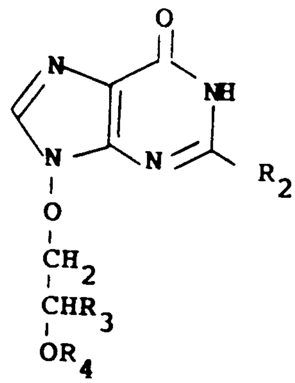

(IA)

The invention also provides a process for the preparation of a compound of formula (I), or a pharmaceutically acceptable salt thereof, which process comprises either

i) imidazole ring closure of a compound of formula II):

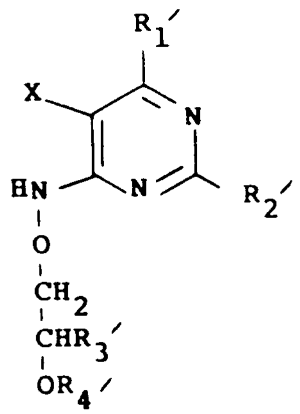

(II)

wherein X is a group capable of cyclising to form an imidazole ring, such as amino or an amino derivative, for example, formylamino; or

ii) pyrimidine ring closure of a compound of formula (III):

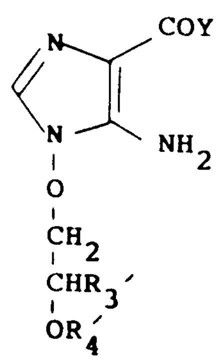

(III)

wherein Y is amino or $C_{1-6}$ alkoxy, with a condensing agent capable of cyclising to form a pyrimidine ring having a 2-$R_2'$ substituent, to give a compound of formula (I) wherein $R_1$ is hydroxy and $R_2$ is amino; or

iii) condensing a compound of formula (IV):

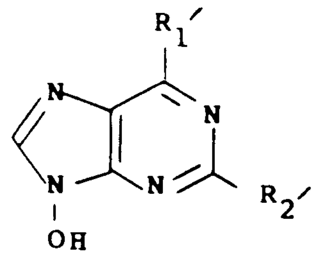

(IV)

with a side chain intermediate of formula (V):

$ZCH_2$-$CHR_3'OR_4'$ (V)

wherein Z is a leaving group;

and wherein, in formulae (II) to (V), $R_1'$, $R_2'$, $R_3'$, $R_4'$ are $R_1$, $R_2$, $R_3$ and $R_4$ respectively, or groups or atoms convertible thereto; and thereafter, when desired or necessary, converting $R_1'$, $R_2'$, $R_3'$ and/or $R_4'$, when other than $R_1$, $R_2$, $R_3$ and/or $R_4$ to $R_1$, $R_2$, $R_3$ and/or $R_4$ respectively, and/or converting $R_1'$, $R_2'$, $R_3'$ and/or $R_4'$ when $R_1$, $R_2$, $R_3$ and/or $R_4$, to other $R_1$, $R_2$, $R_3$ and/or $R_4$.

Process i) may be carried out, preferably when X is formyl, using a cyclisation condensing agent, such as diethoxymethyl acetate or triethyl orthoformate, or by fusion.

Process ii) is preferably carried out in accordance with the methods described in EP-A-242482.

Process iii) may be carried out with suitable values for Z including hydroxy and halo, such as chloro, bromo and iodo, preferably iodo; or other groups readily displaceable by nucleophiles, such as mesyloxy or tosyloxy. The reaction preferably takes place in an inert solvent, such as dimethylformamide at 0-50 °C, preferably ambient temperature. When Z is hydroxy, the reaction takes place in the presence of a dehydrating catalyst, such as diethyl azodicarboxylate in the presence of triphenylphosphine. When Z is halo, the reaction preferably takes place in the presence of a base, such as potassium carbonate.

Examples of conversions of variable groups are as follows:

$R_1'$-$R_1$

a) An $R_1$ hydroxy group may be converted to $R_1'$ is chloro, by chlorination using a reagent such as phosphorus oxychloride, preferably in the presence of tetraethylammonium chloride and dimethylaniline (as acid acceptor) in $CH_3CN$ at reflux temperatures, according to the method described by M.J. Robins and B. Ozanski Can. J. Chem, 59, 2601 (1981).

b) An $R_1'$ chloro group may be converted to $R_1$ is hydroxy by hydrolysis using aqueous mineral acid, such as hydrochloric acid, or more preferably, using an organic acid, such as formic acid at elevated temperature, suitably 70-150° preferably around 100 °C.

c) An $R_1'$ chloro group may be converted to $R_1$ is amino by treatment with ammonia in a lower alkanol, such as ethanol or methanol in an autoclave at 100 °C for a period of about 7 hours, or alternatively, by treatment with sodium azide in dimethylformamide (forming an $R_1$ is $N_3$ intermediate), followed by reduction with ammonium formate/palladium or charcoal, in methanol.

d) An $R_1'$ alkoxy group, such as methoxy, may be converted to $R_1$ hydroxy by the methods of D.R. Haines, J. Med. Chem. 1987, 30 943 and K.K. Ogilvie and H.R. Hanna, Can. J. Chem. 1984, 62, 2702.

e) An $R_1'$ protected amino group, such as tritylamino may be converted to amino, by treatment with aqueous acetic acid, preferably 80% acetic acid at elevated temperature, around 80 °C. $R_1'$ may also be phthalimido, which may be converted to amino by treatment with methyl hydrazine or hydrazine in an inert solvent, such as dichloromethane, at ambient temperature.

$R_2'$-$R_2$

a) $R_2'$ may be protected amino, such as formylamino, which may be converted to $R_2$ is amino by hydrolysis.

$R_3'$-$R_3$

a) $R_3$ hydroxymethyl may be converted to $R_3$ acyloxymethyl by conventional acylation procedures.
b) $R_3'$ may be protected hydroxymethyl, which may be converted to $R_3$ hydroxymethyl by conventional deprotection methods.

Suitable examples of protecting groups and their removal, are as described in EP-A-242482. Particularly suitable protecting groups include the benzyl group, removed by catalytic hydrogenation using palladium/charcoal, 80% acetic acid; the acetate group removed by acid hydrolysis, 2M HCl in ethanol; or the t-butyldimethylsilyl group removable by 80% acetic acid at elevated temperature, around 90 °C.

$R_4'$-$R_4$

a) When $R_5$ and $R_6$ in $R_4$ are other than hydrogen, they may be converted to $R_5$ and $R_6$ are hydrogen, using a deesterifying reagent, such as trimethylsilyl bromide in an aprotic solvent such as dichloromethane or dimethylformamide at ambient temperature, as described by C.E. McKenna et. al. J.C.S. Chem. Comm., 1979, 739.

Selective conversion of one $R_5$ and $R_6$ to hydrogen, may be achieved by treatment with hydroxide ion, as described by Rabinowitz JACS 1960, 82, 4564.

b) $R_4'$ may be hydrogen, which may be converted to $R_4$, by treatment with $QR_4$ wherein Q is a leaving group and $R_4$ is as defined. Q is preferably a tosyloxy group. Conditions for this reaction are i) preliminary formation of an alkoxide using a base, such as sodium hydride, in an aprotic solvent for example dimethylformamide ii) reaction with $QR_4$ at around ambient temperature. The reaction is as described A. Holy et. al. Collect. Czech. Chem. Comm. 1982, 47, 3447.

In this case, $R_5$ and $R_6$ are preferably other than hydrogen.

c) $R_4'$ may be hydrogen, which may be converted, when $R_3$ is hydroxymethyl, to $R_4$ is $CH_2PO(OH)(OR_5)$, by treatment with $ClCH_2PCl_2$ followed by treatment with a base, followed by $OR_5^-$, according to the method described by A. Holy et. al. Czech. Chem. Comm. 1985, 50, 1507; ibid 1987, 52, 2775.

It will be appreciated that the above conversions may take place in any desired or necessary order, having regard to the final desired compound of formula (I).

Intermediates of formula (II) may be prepared from a corresponding compound of formula (VI):

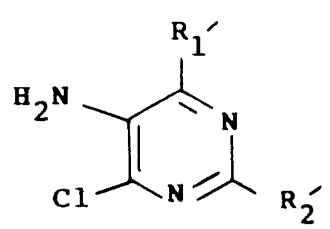

(VI)

and via intermediates of formula (V) wherein Z is OH, as hereinbefore defined, according to the methods described in EP-A-242482 i.e. by converting the compound of formula (V) wherein Z is OH to the phthalimidooxy derivative followed by reaction with methylhydrazine, as described in Descriptions 1 and 4 hereinafter.

The compound of formula (VI) wherein $R_1'$ is chloro and $R_2$ is amino, is a known compound as described by Temple et. al. J. Org. Chem., 40 (21), 3141, 1975.

The compound of formula (VI) wherein $R_1'$ is chloro and $R_2$ is hydrogen is a commercially available compound.

Intermediates of formula (III) may be prepared according to the methods described in EP-A-242482.

Compounds of the formula (IV) are prepared from compounds of formula (VI) wherein the 5-amino group is formylated, by reaction with $R_9ONH_2$ wherein $R_9$ is a protecting group, to give a compound of formula (VII):

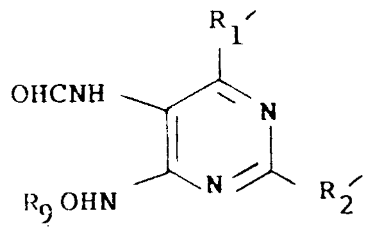

(VII)

which may be cyclised with diethoxymethyl acetate, to give a compound of formula (IV) wherein the OH group is protected. Suitable values for $R_9$ include benzyl, removable by hydrogenation, and the tetrahydropyran-2-yl group removable by treatment with 80% acetic acid, at ambient temperature.

Intermediates of formula (V) wherein Z is hydroxy are known compounds or are prepared by analogous methods to those used for structurally similar known compounds.

It will be appreciated that, when $R_3$ is hydroxymethyl or acyloxymethyl in the resulting compound of formula (I), synthesis of the intermediate of formula (V) wherein Z is hydroxy may involve selective deprotection of an intermediate wherein Z is protected hydroxy and $R_3$ is protected hydroxymethyl, for example, as described in Description 4(b) hereinafter.

Intermediates of formulae (II), (III) and (V) but wherein Z is replaced by an aminooxy group, and wherein $R_4'$ is $R_4$ as defined in formula (I), are believed to be novel and form an aspect of the invention.

Intermediates of the formula (IV) wherein $R_1'$ is chloro and $R_2'$ is hydrogen are also believed to be novel and form an aspect of the invention.

Pharmaceutically acceptable salts may be prepared in conventional manner, for example, in the case of acid addition salts, by reaction with the appropriate organic or inorganic acid.

It will be appreciated that the invention provides a process for the preparation of a compound of formula (I) wherein $R_3$ is hydroxymethyl which process comprises the deprotection of a compound of formula (I) wherein an OH group in $R_3$ is in protected form. Preferred methods for deprotection, as hereinbefore described include removal of the benzyl, acetate or $^t$butyldimethylsilyl group.

The invention also provides a process for the preparation of a compound for formula (I) wherein $R_5$ and $R_6$ are both hydrogen, which process comprises the deesterification of a corresponding compound of formula (I) wherein $R_5$ and $R_6$ are the same alkyl or optionally substituted phenyl group.

The compounds of the invention are of potential use in the treatment of infections caused by viruses, especially herpes viruses such as herpes simplex type 1, herpes simplex type 2; varicella-zoster virus, Epstein-Barr virus and cytomegalovirus; and lentiviruses such as visna virus and human immunodeficiency virus.

The compounds may also be inhibitors of tumorogenic viruses and/or of potential use in the treatment of neoplastic diseases, i.e. cancer.

Compounds of the invention may be formulated for use in a pharmaceutical composition. Accordingly, in a further aspect of the invention, there is provided a pharmaceutical composition which comprises a compound of formula (I) or pharmaceutically acceptable salt thereof together with a pharmaceutically acceptable carrier or excipient.

A composition which may be administered by the oral route to humans may be compounded in the form of a syrup, tablet or capsule. When the composition is in the form of a tablet, any pharmaceutical carrier suitable for formulating such solid compositions may be used, for example magnesium stearate, starch, lactose, glucose, rice, flour and chalk. The composition may also be inthe form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the composition may be in the form of a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art, or an ointment composition.

Preferably, the composition of this invention is in unit dosage form or in some other form that may be administered in a single dose. A suitable dosage unit might contain from 50 mg to 1 g of active ingredient, for example 100 to 500 mg.

Such doses may be administered 1 to 4 times a day or more usually 2 or 3 times a day. The effective dose of compound will in general by in the range of from 1.0 to 20 mg/kg of body weight per day or more usually 2.0 to 10 mg/kg per day.

No unacceptable toxicological effects are indicated at the above described dosage levels.

The invention also provides a compound of formula (I) or a pharmaceutically acceptable salt thereof for use as an active therapeutic substance, in particular for the treatment of viral infections.

The compounds of the invention are also believed to exhibit a synergistic antiviral effect in conjunction with interferons; and combination products comprising these two components for sequential or concomitant administration, by the same or different routes, are therefore within the ambit of the present invention.

The following examples illustrate the invention; the following descriptions illustrate the preparation of intermediates.

Description 1 (Intermediates (V) for Example 1 to 4).

(a) Diethyl 2-hydroxyethoxymethylphosphonate

To a solution of diethyl 2-acetoxyethoxymethylphosphonate*(16 g, 63mmol) in absolute ethanol (150ml), was added 0.5M sodium ethoxide (12.19ml). After standing at ambient temperature overnight, 1R-120H resin was added until pH 6.5 was reached. The solution was filtered immediately, the resin washed with ethanol and the combined filtrates evaporated under reduced pressure. The residue was chromatographed on silica gel (eluted with chloroform: methanol, 98:2) to yield the title compound as a colourless oil (9.3g, 70%). IR: $\nu_{max}$ (film) 3420, 3000, 1450, 1395, 1240, 1170, 1130, 1030, 970 $cm^{-1}$. $^1H$ NMR: $\delta_H$ ($CDCl_3$) 1.35 (6H,t,J=7Hz, $(OCH_2C\underline{H}_3)_2$), 3.75 (4H,br.s, $OC\underline{H}_2C\underline{H}_2O$), 3.90 (2H,d,J=8Hz, O-$C\underline{H}_2P$), 4.20 (4H,m,(O-$C\underline{H}_2CH_3)_2$), 5.20 (1H,br.s, $D_2O$ exchangeable, OH).
Found: C,38.64; H,8.27%. $C_7H_{17}PO_5$. $0.3H_2O$
requires: C,38.63; H,8.01%.

(b) Diethyl 2-(N-phthalimidooxy)ethoxymethylphosphonate

Diethyl azodicarboxylate (8.12g, 46.7mmol) was added to a solution of diethyl 2-hydroxyethoxymethylphosphonate (9.0g, 42.4mmol), N-hydroxyphthalimide (6.92g, 42.4mmol) and triphenylphosphine (21.33g, 46.7mmol) in dry tetrahydrofuran (150ml) at ambient temperature, under a nitrogen atmosphere. After 3 days, the mixture was evaporated to dryness, the residue dissolved in diethyl ether and the solution kept at 4°C for 7 days. The cooled mixture was filtered and the filtrate evaporated to dryness. The residue was chromatographed on silica gel (eluted with hexane: ethyl acetate, 50:50 to remove triphenylphosphine oxide, then changed to ethyl acetate) to give the title compound as a light orange coloured oil (10.6g, 70%). IR: $\nu_{max}$ (film) 3510, 3000, 2950, 2920, 1795, 1745, 1735, 1430, 1380, 1250, 1190, 1165, 1130, 1030, 980, 880, 710 $cm^{-1}$. $^1H$ NMR: $\delta_H$ ($CDCl_3$) 1.32 (6H,t,J=7Hz, $(-OCH_2C\underline{H}_3)_2$), 3.93 (2H,d, J=8Hz, O-$C\underline{H}_2P$), 3.98 (2H,m,$CH_2C\underline{H}_2OCH_2P$), 4.16 (4H,m, $(OC\underline{H}_2CH_3)_2$), 4.39 (2H,m,N-$OC\underline{H}_2CH_2$), 7.74-786 (4H,m,phthalyl H).
Found: C,50.65; H,5.94; N,3.76%. $C_{15}H_{20}NO_7P$
requires: C,50.42; H,5.64; N,3.92%.
Found: m/z 358.1042($MH^+$). $C_{15}H_{21}NO_7P$
requires: m/z 358.1056.

c) Diethyl 2-(aminooxy)ethoxymethylphosphonate

To a solution of diethyl 2-(N-phthalimidooxy)ethoxymethylphosphonate (10.25g, 29mmol) in dry dichloromethane (55ml) was added methylhydrazine (1.83ml, 34.4mmol). After stirring at ambient temperature for 2 hours, the reaction mixture was filtered and evaporated to dryness. The residue was chromatographed on silica gel (eluted with dichloromethane: methanol 98:2) to give the title compound as an oil (6g, 92%). IR: $\nu_{max}$ (film) 3480, 3320, 2995, 2920, 1600, 1445, 1390, 1370, 1240, 1170, 970 $cm^{-1}$. $^1H$ NMR: $\delta_H$ ($CDCl_3$) 1.35 (6H,t,J=7Hz, $(OCH_2C\underline{H}_3)_2$), 3.8-3.95 (4H,m, O-$C\underline{H}_2C\underline{H}_2O$), 3.84 (2H,d,J=8Hz, $OC\underline{H}_2P$), 4.21 (4H,dq,J=7Hz, 7Hz, $(OC\underline{H}_2CH_3)_2$), 4.8 (2H,br.s, $D_2O$ exchangeable, $NH_2$).

*Can.J.Chem.60, 547(1982); Chem,. Abs. 55, 15507c 1961

Found: C,36.58; H,7.98; N,6.22%. $C_7H_{18}NO_5P$ requires: C,37.00; H,7.98; N,6.17%.
Found: m/z 228.0987($MH^+$). $C_7H_{19}NO_5P$ requires: m/z 228.1002.

Description 2 (Intermediates for Examples 1 and 2)

a) 4-Chloro-6-[2-(diethoxyphosphorylmethoxy)ethoxyamino]-5-formamidopyrimidine

A mixture of 4,6-dichloro-5-formamidopyrimidine (1.9g, 10mmol), diethyl 2-(aminooxy)-ethoxymethylphosphonate (2.27g, 10mmol) and triethylamine (2ml, 15mmol) in dry dioxan (50ml) was heated at 100°C for 4 hours. After cooling to ambient temperature, the reaction mixture was filtered and the filtrate concentrated under reduced pressure. The residual oil was chromatographed on silica gel (eluted initially with dichloromethane: methanol, 98:2, changed to dichloromethane; methanol, 95:5) to give the title compound as an oil (2.7g, 70%). IR: $\nu_{max}$ (film) 3200, 2995, 1690, 1640, 1600, 1570, 1240, 1165, 1030 $cm^{-1}$. $^1H$ NMR: $\delta_H$ [$(CD_3)_2S0$ + $D_2O$] 1.33 (6H,t,J=7Hz, ($OCH_2C\underline{H_3})_2$), 3.76 (2H,m, -$C\underline{H_2}OCH_2OP$, 3.86 (2H,d,J=8Hz, $OC\underline{H_2}P$), 4.0-4.1 (6H,m, N-$OC\underline{H_2}$ + ($OC\underline{H_2}CH_3)_2$), 8.15 (1H,s,H-2)
Found: C,37.10; H,5.52; N,14.27%; m/z 382.0807($M^+$).
$C_{12}H_{20}N_4O_6PCl$ requires: C,37.66; H,5.27; N,14.64%; m/z 382.0809 ($M^+$).

b) 6-Chloro-9-[2-(diethoxyphosphorylmethoxy)ethoxy]purine

A solution of 4-chloro-6-[2-(diethoxyphosphorylmethoxy)ethoxyamino]-5-formamidopyrimidine (2.4g, 6.3mmol) in diethoxymethyl acetate (10ml) was heated at 120°C for 45 minutes. After cooling to ambient temperature, the solvent was removed under reduced pressure. the residue was dissolved in methanol (15ml) and .880 ammonia (1ml). After 15 minutes, the reaction mixture was evaporated to dryness and the residue chromatographed on silica gel (eluted with dichloromethane: methanol, 98:2) to give the title compound as an oil (2.15g, 94%). IR: $\nu_{max}$ (film) 3100, 3070, 2995, 1600, 1570, 1440, 1395, 1335, 1250, 1220, 1170, 1030, 930, 640 $cm^{-1}$. $^1H$ NMR $\delta_H$ [$(CD_3)_2SO$] 1.23 (6H,t,J=7Hz, ($OCH_2C\underline{H_3})_2$), 3.88 (2H,d, J=8Hz, $OC\underline{H_2}P$), 3.89 (2H,m, $C\underline{H_2}OCH_2P$), 4.0-4.1 (4H,m, ($OC\underline{H_2}CH_3)_2$), 4.61 (2H,m, N-$OC\underline{H_2}$), 8.82 (1H,s,H-8), 8.97 (1H,s,H-2).
Found: C,38.38; H,5.10; N,15.14%.. $C_{12}H_{18}N_4O_5PCl$.
$0.5H_2O$ requires: C,38.56; H,5.12; N,14.99%.
Found: m/z 364.0703.
$C_{12}H_{18}N_4O_5PCl$ requires: m/z 364.0701.

Description 3 (Intermediates for Examples 3, 4 (Method B) and 5)

a) 4-Chloro-6-[2-(diethoxyphosphorylmethoxy)ethoxyamino]-2,5-diformamidopyrimidine

A mixture of 4,6-dichloro-2,5-diformamidopyrimidine (2.3g, 10 mmol), diethyl 2-(aminooxy)-ethoxymethylphosphonate (2.27g, 10 mmol), and diisopropylethylamine (3.48ml, 20 mmol) in diglyme (40 ml) was heated at 100°C for 3 hours. After cooling to ambient temperature, the solvent was evaporated under reduced pressure and the residue obtained chromatographed on silica gel (eluted with dichloromethane: methanol 97.3) to give the title compound as a yellow foam (2.8g, 65%).
$^1H$ NMR: $\delta_H$ [$(DC_3)_2SO$] 1.26 (6H, t, J=7Hz, ($OCH_2C\underline{H_3})_2$), 3.76 (2H, m $C\underline{H_2}OCH_2P$), 3.88 (2H, d, J=8Hz, $OC\underline{H_2}P$), 3.95-4.10 (6H, m, N-$OC\underline{H_2}$ + (O-$C\underline{H_2}CH_3)_2$), 8.14 (1H, s, NHC$\underline{H}$O), 9.16 + 9.41 (combined 1H, $D_2O$ exchangeable N$\underline{H}$CHO), 9.24 (1H, br.s, NHC$\underline{H}$O), 10.85 (2H, br.s, $D_2O$ exchangeable, N$\underline{H}$$OCH_2$ + N$\underline{H}$CHO).

b) 6-Chloro-9-[2-(diethoxyphosphorylmethoxy)ethoxy]-2-formamidopurine

A solution of 4-chloro-6-[2-(diethoxyphosphorylmethoxy) ethoxyamino]-2,5-diformamidopyrimidine (2.8g, 6.6 mmol) in diethoxymethyl acetate (20 ml) was heated at 120°C for 2 hours. After cooling to ambient temperature, excess solvent was removed under reduced pressure, the residue dissolved in methanol (20 ml) and .880 ammonia (6.5 ml) and the solution stirred at ambient temperature for 1 hour. The solvent was evaporated to leave an oil which was chromatographed on silica gel (eluted with dichloromethane: methanol, 98.2) to give the title compound as a pale yellow oil (2.4g, 90%). IR: $\nu_{max}$ (film) 3480, 3120, 2995, 1710,

1610, 1580, 1510, 1440, 1390, 1330, 1240, 1050, 1030, 970, 920, 780 $cm^{-1}$.
$^1$H NMR: $\delta_H$ [$(CD_3)_2SO$] 1.23 (6H, t, J = 7 Hz, $(OCH_2CH_3)_2$), 3.87 (2H, d, J = 8 Hz, $OCH_2P$), 3.87 (2H, m, N-$OCH_2CH_2O$), 4.03 (4H, m, $(OCH_2CH_3)_2$), 4.56 (2H, m, $NOCH_2CH_2$), 8.75 (1H, s, H-8), 9.38 (1H, br.s, CHO), 11.30 (1H, br.s, $D_2O$exchangeable NHCHO).
Found: C, 38.19; H, 4.79; N, 16.74%. $C_{13}H_{19}N_5O_6PCl$
requires: C, 38.29; H, 4.70; N, 17.17%.
Found: m/z 408.0827 ($MH^+$). $C_{13}H_{20}N_5O_6PCl$ requires: m/z 408.0840 ($MH^+$).

Description 4 (Intermediates (V) for Examples 6 (Method B), 7, 8, 9 (Method A) and 10)

a) Diethyl [2-benzyloxy-1-(benzyloxymethyl)ethoxy]methylphosphonate

Dry HCl, gas was bubbled through an ice-cooled solution of 1,3-dibenzyloxypropan-2-ol (25g, 0.092mol) and paraformaldehyde (2.75g, 0.092mol) in dry dichloromethane (100ml) for 1 hour. The resulting solution was dried ($MgSO_4$) and evaporated to dryness to leave an oil. Triethyl phosphite (15.7ml, 0.092mol) was added and the resulting mixture stirred and heated at 140°C for 16 hours. The liquid obtained was dissolved in ethyl acetate and washed with sodium bicarbonate solution. The organic phase was washed with brine, dried ($MgSO_4$) and evaporated to give a mobile oil, which was chromatographed in silica (hexane/ethyl acetate 50:50 as eluant) to give the title compound as a colourless, mobile oil (19.7g, 51%). IR:$\nu_{max}$ (film) 3055, 3020, 2900, 2860, 1490, 1470, 1450, 1390, 1305, 1255, 1095, 1050, 1030, 970, 820, 770, 735, 700 $cm^{-1}$. $^1$H NMR: $\delta_H$ ($CDCl_3$) 1.3 (6H, t, J = 7Hz, P-$(OCH_2CH_3)$), 3.6 (4H, d, 2x$CH_2OCH_2Ph$), 3.8-4.05 (1H, m, CH), 4.05 (2H, d, J = 9Hz, $OCH_2P$), 4.0-4.3 (4H, m, P-$(OCH_2CH_3)_2$), 4.55 (4H, s, 2 x $OCH_2Ph$), 7.38 (10H, s, 2 x Ph). $C_{22}H_{31}PO_6$ requires: C, 62.54; H, 7.42 Found: C, 62.75, H, 7.61. m/z: $C_{22}H_{31}PO_6$ requires 422.1858; observed ($M^+$) 422.1864.

b) Diethyl [2-hydroxy-1-(hydroxymethyl)ethoxy]methylphosphonate and Diethyl [2-benzyloxy-1-(hydroxymethyl)ethoxy]methylphosphonate

To a solution of diethyl [2-benzyloxy-1-(benzyloxymethyl)ethoxy]methylphosphonate (15.5g, 36.7mmol) in ethanol (200ml), containing a trace of methanolic HCl (1ml), was added 10% Pd-C (1.5g). The mixture was treated with hydrogen at atmospheric temperature and pressure for a total of 6 days. Filtration and evaporation of the solvent gave an oil which was chromotagraphed on silica (eluant dichloromethane/methanol 95:5, then 92:8) after which were obtained the monobenzyl ether (3.65g, 30%) and the diol (2.73g, 30%).

Data for diol. IR: $\nu_{max}$ (film) 3400, 2980, 2940, 2920, 1650, 1480, 1445, 1390, 1370, 1295, 1230, 1165, 1120, 1020, 980, 880, 820, 780 $cm^{-1}$. $^1$H NMR: $\delta_H$ [$(CD_3)_2SO$] 1.23 (6H, t, J = 7Hz, $(OCH_2CH_3)_2$), 3.25-3.6 (5H, m, $CH_2CHCH_2$), 3.94 (2H, d, J = 8.2Hz, $OCH_2P$), 4.0 - 4.2 (4H, m, $(OCH_2CH_3)_2$), 4.56 (2H, br.s, $D_2O$ exchangeable, 2 x OH). m/z: $C_8H_{20}O_6P$ requires: 243.0998; observed 243.0986 (M + $H^+$).

Data for monobenzyl ether. IR: $\nu_{max}$ (film) 3395, 2980, 2900, 2860, 1475, 1450, 1390, 1365, 1240, 1160, 1090, 1050, 1020, 970, 810, 775, 730, 695 $cm^{-1}$. $^1$H NMR: $\delta_H$[$(CD_3)_2SO$] 1.21, 1.20 (6H, 2 x t, J = 7Hz, 7Hz, (0-$CH_2CH_3)_2$) 3.5 - 3.7 (5H, M, $CH_2CHCH_2$-), 3.95 (2H, d, d, J = 8.8HZ, $OCH_2P$), 4.O (4H, M, $OCH_2CH_3)_2$), 4.49 (2H, s, $OCH_2Ph$), 4.68 (1H, t, $D_2O$ exchangeable OH), 7.2 - 7.5 (5H, m, Ph). $C_{15}H_{25}O_6P$. O.4 $H_2O$ requires: C, 53.06; H, 7.67%. Found: C, 53.11; H, 7.94%. m/z: $C_{15}H_{26}O_6P$ requires 333.1467, observed: 333.1472 (M + $H^+$).

c) Diethyl [2-benzyloxy-1-(N-phthalimidooxymethyl)ethoxy]methylphosphonate

Diethyl azodicarboxylate (1.82ml, 11.6mmol) was added to a solution of diethyl [2-benzyloxy-1-(hydroxymethyl) ethoxy]methylphosphonate (3.5g, 10.5 mmol), N-hydroxyphthalimide (1.72g, 10.5mmol) and triphenylphosphine (3.04g, 11.6mmol) in dry THF (60ml). The reaction mixture was left at ambient temperature for 5 days, then evaporated to dryness. The residue obtained was dissolved in diethyl ether and left at 5°C for 16 hours. The precipitated crystals of triphenylphosphine oxide were filtered off and the ether filtrate evaporated to dryness to leave an oil. After chromatography on silica gel (eluant hexane/ethyl acetate 50:50, then 30:70, then 20:80) the title compound was obtained as a colourless oil (4.1g, 80%). IR: $\nu_{max}$ (film) 3060, 3020, 2980, 2930, 2920, 1790, 1740, 1465, 1450, 1370, 1250, 1190, 1160, 1130, 1100, 1080, 1050, 1030, 970, 870, 820, 780, 740, 700$cm^{-1}$. $^1$H NMR: $\delta_H$ ($CDCl_3$) 1.30 (6H, t, J = 7Hz, $(OCH_2CH_3)_2$), 3.7 (2H, d, J = 5Hz, $CHCH_2OCH_2Ph$), 4.0-4.25 (7H, m, $(OCH_2CH_3)_2$, $OCH_2Ph$, $CH_2CHCH_2$), 4.36 (2H, m, $CH_2ON$), 4.56 (2H, s, $OCH_2Ph$), 7.30 - 7.35 (5H, br.s, Ph), 7.74 - 7.9 (4H, m, phthalimide aromatic H). m/z:

$C_{23}H_{28}NO_8P$ requires 477.1553; observed: 477.1548 ($M^+$).

d) Diethyl (1-aminooxymethyl-2-benzyloxyethoxy)methylphosphonate

To a solution of diethyl [2-benzyloxy-1-(N-phthalimidooxymethyl)ethoxy]methylphosphonate, (4g, 8.4mmol) in dry dichloromethane (40ml), was added methylhydrazine (0,54ml, 10.1mmol) at ambient temperature and the mixture stirred for 2 hours. The solution was filtered and the filtrate evaporated to dryness. The residue was dissolved in ether and left at 5°C overnight. The deposited solid was filtered off, the filtrate evaporated and the residue chromatographed on silica (eluant dichoromethane/methanol 98:2) to give the title compound as a colourless oil (2.7g, 92%).
$^1H$ NMR: $\delta_H$ ($CDCl_3$) 1.32, 1.33 (6H, 2xt, P-($OCH_2CH_3)_2$), 3.5-3.65 (2H, m, $CH_2ONH_2$), 3.78 (2H, d, $CH_2OCH_2PH$), 3.95 - 4.1 (3H, m, $OCH_2P$ + $CH_2CHCH_2$), 4.1 - 4.3 (4H, m, P-($OCH_2CH_3)_2$), 4.53 (2H, s, $OCH_2Ph$), 5.0 - 6.0 (2H, br.s, $D_2O$ exchangeable, $NH_2$), 7.27 - 7.35 (5H, m, aromatic H). m/z: $C_{15}H_{26}NO_6P$ requires 347.1498; observed 347.1516 ($M^+$).

Description 5 (Intermediates for Examples 6 (Method B), 7 and 8)

a) 6-[3-Benzyloxy-2-(diethoxyphosphorylmethoxy)propoxyamino]-4-chloro-5-formidopyrimidine

A mixture of 4,6-dichloro-5-formamidopyrimidine (390mg, 2mmol), diethyl (1-aminooxymethyl-2-benzyloxyethoxy)methylphosphonate (700mg, 2mmol) and triethylamine (0.41ml, 3mmol) in dry dioxan (5ml) was heated at 100°C for 2 hours. The reaction mixture was cooled, filtered and the filtrate evaporated to leave a yellow oil which was chromatographed on silica (dichloromethane/methanol 98:2 as eluant) to give the title compound as a yellow viscous oil (750mg, 74%), $^1H$ NMR: $\delta_H$ [$(CD_3)_2SO + D_2O$] 1.30 (6H, t, J=7Hz, ($OCH_2CH_3)_2$), 3.55-3.8 (2H, m, $CH_2OCH_2Ph$), 3.9-4.2 (9H, m, ($OCH_2CH_3)_2$ + $OCH_2P$ + $NHOCH_2CH$), 4.6 (2H, s, $OCH_2Ph$), 7.42 (m, 5H, aromatic Ph), 8.24 (1H, s, H-2). Found: C, 47.66; H,5.69; N, 10.51%. $C_{26}H_{28}N_4O_7PCl$ requires: C, 47.76; H, 5.61; N,11.14%, m/z: $C_{20}H_{28}N_4O_7PCl$ requires: 502.1384; observed: 502.1340 ($M^+$).

b) 9-[3-Benzyloxy-2-(diethoxyphosphorylmethoxy)propoxy]-6-chloropurine

A solution of 6-[3-benzyloxy-2-(diethoxyphosphorylmethoxy)propoxyamino]-4-chloro-5-formamidopyrimidine (750mg, 1.49mmol) in diethoxymethyl acetate (2ml) was stirred and heated at 120°C for 2 hours. After cooling to ambient temperature, the excess solvent was evaporated. The residue was dissolved in methanol (10ml) and .880 ammonia (1ml) and left at ambient temperature for 10 minutes. The solvent was removed under reduced pressure and the residue chromatographed on silica (dichloromethane/methanol, 98:2 as eluent) to give the title compound as a yellow oil (590mg, 80%). IR: $\nu_{max}$ - (film) 3060, 2990, 2905, 1590, 1565, 1435, 1330, 1250, 1220, 1165, 1100, 1050, 1030, 970, 930, 850 $cm^{-1}$. $^1H$ NMR: $\delta_H$ [$(CD_3)_2SO$] 1.21 (6H, t, J=7Hz, ($OCH_2CH_3)_2$), 3.6-3.75 (2H, m, $CH_2OCH_2Ph$), 3.8-4.1 (7H, m, ($OCH_2CH_3)_2$ + $OCH_2P$ + $CH_2CHCH_2$), 4.53 (2H, s, $OCH_2Ph$), 4.5-4.7 (2H, m, N-$OCH_2$), 7.32 (5H, br.s, Ph), 8.82 (1H, s), 9.02 (1H, s) Found: C, 49.71; H, 5.68; N, 10.94%. $C_{20}H_{26}N_4O_6PCl$ requires: C, 49.54; H, 5.40; N, 11.56%. m/z: $C_{20}H_{26}N_4O_6PCl$ requires 484.1279; observed 484.1249 ($M^+$).

c) 9-[3-Benzyloxy-2-(diethoxyphosphorylmethoxy)propoxy]adenine

A solution of 9-[3-benzyloxy-2-(diethoxyphosphorylmethoxy)propoxy]-6-chloropurine (570mg, 1.18mmol) in ethanolic ammonia (10ml) was heated in a sealed vessel at 110°C for 2 hours. After cooling to ambient temperature, the solvent was evaporated and the residue obtained chromatographed on silica (dichloromethane/methanol 95:5 as eluant) to give the title compound (350mg, 64%). IR: $\nu_{max}$ (film) 3320, 3200, 2980, 2900, 1640, 1595, 1470, 1450, 1410, 1390, 1370, 1330, 1290, 1240, 1160, 1090, 1050, 1020, 970, 820, 790, 730, 700, $cm^{-1}$. $^1H$ NMR: $\delta_H$ [$(CD_3)_2SO$] 1.22 (6H, t, J = 7Hz, ($OCH_2CH_3)_2$), 3.6-3.75 (2H, m, $CH_2OCH_2Ph$), 3.95-4.2 (7H, m, ($OCH_2CH_3)_2$ + $CH_2CHCH_2$ + $OCH_2P$), 4.4-4.65 (2H, m, N-$OCH_2$), 4.52 (2H, s, $OCH_2Ph$), 7.25-7.4 (5H, m, aromatic), 7.39 (2H, br.s, $D_2O$ exchangeable $NH_2$), 8.15(1H, s), 8.41 (1H, s). Found: C, 49.81; H, 5.75; N, 14.07%; $C_{20}H_{28}N_5O_6P$. $H_20$ requires: C, 49.68; H, 6.25; N, 14.48%, m/z: $C_{20}H_{28}N_5O_5P$ requires 465.1777; observed: 467.1757.

Description 6 (Intermediates for Examples 9 (Method A) and 10)

a) 6-[3-Benzyloxy-2-(diethoxyphosphorylmethoxy)propoxyamino]-4-chloro-2,5-diformamidopyrimidine

A mixture of 4,6-dichloro-2-5-diformamidopyrimidine (650mg, 2.74mmol), diethyl (1-aminooxymethyl-2-benzyloxyethoxy)methylphosphonate (950mg, 2.74 mmol) and diisopropylethylamine (0.95ml, 5.5mmol) in diglyme (12ml) was heated at 100°C for 2½ hours. The mixture was cooled and the solvent evaporated to leave an oily residue which was chromatographed on silica (dichloromethane/methanol 97:3 as eluant) to give the title compound as a yellow foam (800mg, 54%). $^1$H NMR: $\delta_H$ [$(CD_3)_2SO$] 1.21 (6H, 2 x t, P-$(OCH_2C\underline{H}_3)_2$), 3.5-3.7 (2H, m, $C\underline{H}_2OCH_2Ph$), 3.75-4.2 (9H, m, $(OC\underline{H}_2CH_3)_2$ + $OC\underline{H}_2C\underline{H}$ + $OC\underline{H}_2P$), 4.51 (2H, s, $OC\underline{H}_2Ph$), 7.25-7.5 (5H, m, $P\underline{h}$), 8.15 (1H, s, $C\underline{H}O$), 9.26 (1H, br.s, $C\underline{H}O$), 9.44-10.88 (3H, 2 x br.s, $D_2O$ exchangeable $N\underline{H}$ + 2 x $N\underline{H}CHO$). Found: C, 46.32; H, 5.61; N, 11.94%. $C_{21}H_{29}N_5O_8PCl$ requires: C, 46.20; H, 5.35; N, 12.83%. m/z: 545 ($M^+$).

b) 9-[3-Benzyloxy-2-(diethoxyphosphorylmethoxy)propoxy]-6-chloro-2-formamidopurine

A solution of 6-[3-benzyloxy-2-(diethoxyphosphorylmethoxy)propoxyamino]-4-chloro-2,5-diformamidopyrimidine (760mg, 1.4mmol) in diethoxymethyl acetate (2ml) was stirred and heated in an oil bath at 120°C for 2 hours. After cooling to ambient temperature, the solvent was evaporated and the residue dissolved in methanol (10ml) and .880 ammonia (1ml). After 15 minutes at ambient temperature the solution was evaporated to dryness. The residue obtained was chromatographed on silica gel (dichloromethane/methanol, 98:2 as eluant) to give the title compound as a colourless oil (550mg, 75%). IR: $\nu_{max}$ (film), 3400, 3220, 3110, 2980, 2900, 1710, 1610, 1580, 1510, 1475, 1440, 1385, 1330, 1240, 1160, 1140, 1095, 1050, 1020, 980, 920, 820, 780, 740, 700 cm$^{-1}$. $^1$H NMR: $\delta_H$ [$(CD_3)_2SO$] 1.20, 1.21 (6H, 2 x t, P-$(OCH_2C\underline{H}_3)_2$), 3.6-3.75 (2H, m, $CHC\underline{H}_2$), 3.8-4.15 (7H, m, $CH_2C\underline{H}CH_2$, $OC\underline{H}_2P$, P-$(OC\underline{H}_2CH_3)_2$), 4.52 (2H, s, $OC\underline{H}_2Ph$), 4.45-4.65 (2H, m, N-$OC\underline{H}_2$), 7.25-7.40 (5H, m, Ph), 8.81 (1H, s, H-8), 9.37 (1H, br.s, $NHC\underline{H}O$), 11.31 (1H, br.s, $D_2O$ exchangeable $NHC\underline{H}O$). Found: C, 48.11; H, 5.37; N, 12.50%. $C_{21}H_{27}N_5O_7PCl$ requires: 47.77; H, 5.16; N, 13.27%. m/z: $C_{21}H_{28}N_5O_7PCl$ requires 528.1415; observed 528.1363 (M + $H^+$).

c) 9-[3-Benzyloxy-2-(diethoxyphosphorylmethoxy)propoxy]quanine

A solution of 9-[3-benzyloxy-2-(diethoxyphosphorylmethoxy)propoxy]-6-chloro-2-formamidopurine (520mg, 1mmol) in 80% aqueous formic acid (6ml) was stirred and heated at 80°C for 5 hours. After cooling and evaporation to dryness, the residue was dissolved in methanol (5ml) and .880 ammonia (1ml). After 15 minutes at ambient temperature, the solvent was evaporated and the residue obtained chromatographed on silica gel (dichloromethane/methanol 90:10 as eluant) to give the title compound as a white solid (330mg, 70%), mp. 166-169° (acetone). IR: $\nu_{max}$ (KBr) 3326, 3167, 2983, 2907, 2868, 2745, 1694, 1648, 1600, 1586, 1540, 1475, 1454, 1391, 1328, 1251, 1163, 1100, 1051, 1027, 970, 823, 787, 739, 693, 624, cm$^{-1}$. UV: $\lambda_{max}$ (EtOH) 255nm (14,100), $^1$H NMR: $\delta_H$ [$(CD_3)_2SO$], 1.22 (6H, 2 x t, J=7Hz, P-$(OCH_2C\underline{H}_3)_2$), 3.5-3.8 (2H, m, $C\underline{H}_2OCH_2Ph$), 3.85-4.25 (7H, m, $OC\underline{H}_2P$, P-$(OC\underline{H}_2CH_3)_2$, $CH_2C\underline{H}CH_2$), 4.3-4.5 (2H, m, N-$OC\underline{H}_2$), 4.50 (2H, s, $OC\underline{H}_2Ph$), 6.58 (2H, br.s, $D_2O$ exchangeable $N\underline{H}_2$), 7.25-7.5 (5H, m, Ph), 7.95 (1H, s, H-8), 10.69 (1H, br.s, $D_2O$ exchangeable $N\underline{H}$). Found: C, 49.35; H, 5.85; N, 14.60%. $C_{20}H_{28}N_5O_7P$. $0.25H_2O$ requires: C, 49.43; H, 5.91; N, 14.41%. m/z: (FAB, matrix thioglycerol)482 (M+$H^+$).

Description 7 (Intermediates (V) for Examples 6 (Methods A and C), 7, 8, 9 (Method B), 10, 15 and 16)

a) Diethyl [2-acetoxy-1-(hydroxymethyl)ethoxy]methylphosphonate

To a solution of diethyl [2-hydroxy-1-(hydroxymethyl)ethoxy]methylphosphonate (6.1g, 25.2mmol) in dry THF (75ml) were added p-toluenesulphonic acid (250mg) and trimethyl orthoacetate (4.45ml, 35mmol). The reaction mixture was stirred at ambient temperature for 16 hours after which water (1ml) plus 2MHCl (5 drops) were added. After stirring for a further 30 minutes, the solution was evaporated to dryness and the residue chromatographed on silica gel (eluant dichloromethane: methanol 97:3) to give the title compound (5.1g, 71%) as colourless mobile oil. IR: $\nu_{max}$ (film) 3400, 2980, 2920, 2910, 1740, 1440, 1390, 1370, 1245, 1160, 1120, 1050, 1030, 970, 820, 780cm$^{-1}$. $^1$H NMR: $\delta_H$ ($CDCl_3$) 1.36 (6H, t, J=7Hz, $P(OCH_2C\underline{H}_3)_2$), 2.10 (3H,s, $COCH_3$), 3.65-4.50 (12H,m, $P(OC\underline{H}_2C\underline{H}_3)_2$, $OC\underline{H}_2P$, $OC\underline{H}_2C\underline{H}C\underline{H}_2O\underline{H}$). Found: C,41.79; H,7.66%. $C_{10}H_{21}O_7P$ requires: C,42.25; H,7.66%. m/z: (Isobutane C.I) 285 ($MH^+$, 100%).

b) Diethyl [2-acetoxy-1-(N-phthalmidooxymethyl)ethoxy]methylphosphonate

Diethyl azodicarboxylate (1.89ml, 12mmol) was added to a solution of diethyl [2-acetoxy-1-(hydroxymethyl)ethoxy] methylphosphonate (3.1g, 10.9mmol), triphenylphosphine (3.15g, 12mmol) and N-hydroxyphthalimide (1.78g, 10.7mmol) in dry THF (60ml). The reaction mixture was stirred at ambient temperature under an atmosphere of nitrogen for 16 hours, and then evaporated to dryness. The residue obtained was dissolved in diethyl ether and left at 4°C for 16 hours. The crystals of triphenylphosphine oxide were removed by filtration and the filtrate evaporated to dryness. The residue was chromatographed on silica gel (eluant hexane:acetone 70:30) to give the title compound as a pale yellow oil (3.6g, 76%). IR: $\nu_{max}$ (film) 3500, 3460, 2980, 2900, 1780, 1735, 1465, 1440, 1370, 1240, 1185, 1160, 1030, 1020, 970, 875, 820, 780, 700cm$^{-1}$. $^1$H NMR: $\delta_H$ ($CDCl_3$) 1.33 (6H,2 x t, J=7Hz, ($OCH_2C\underline{H}_3)_2$, 2.09 (3H,s, $COCH_3$), 3.9-4.5 (11H,m, ($OC\underline{H}_2CH_3)_2$, + $OC\underline{H}_2P$, + $OC\underline{H}_2C\underline{H}C\underline{H}_2$), 7.75-7.87 (4H,m, aromatic H). Found: C,50.14; H,5.69%; N, 3.15%. $C_{18}H_{24}NO_9P$ requires: C,50.35; H,5.63; N, 3.26%. m/z: observed 430.1275; $C_{18}H_{25}NO_9P$ ($MH^+$) requires 430.1267.

c) Diethyl [2-acetoxy-1-(aminooxymethyl)ethoxy]methylphosphonate

To a solution of diethyl [2-acetoxy-1-(N-phthalimidooxymethyl)ethoxy]methylphosphonate (1.0g, 2.33m mol), in dry dichloromethane (20ml) was added methylhydrazine (0.125ml, 2.35mmol) and the solution stirred at ambient temperature for 10 minutes. The reaction mixture was filtered, and the filtrate evaporated to dryness. The residue obtained was chromatographed on silica gel (eluant dichloromethane methanol 98:2) to give the title compound as a colourless oil (0.59g, 86%). IR: $\nu_{max}$ (film) 3460, 3320, 3240, 3170, 2995, 2910, 1740, 1595, 1445, 1390, 1370, 1240, 1165, 1115, 1050, 1025, 975, 820, 780cm$^{-1}$. $^1$H NMR: $\delta_H$ - [$(CD_3)_2SO$] 1.23 (6H, t, J=7Hz, $(OCH_2C\underline{H}_3)_2$), 2.02 (3H,s, $COCH_3$), 3.5-3.65 (2H,m, $NOCH_2$), 3.8-4.25 (9H,m, $OC\underline{H}_2P$) $(OC\underline{H}_2CH_3)_2$ + $C\underline{H}C\underline{H}_2$), 6.13 (2H br. s, $D_2O$ exchangeable $NH_2$). Found: C,39.52; H,7.20; N,4.46%; $C_{10}H_{22}NO_7P$ requires: C,40.13; H,7.41; N, 4.68%. m/z: observed 299.1117; $C_{10}H_{22}NO_7P$ ($M^+$) requires 299.1134.

Description 8 (Intermediates for Examples 6 (Method A), 7 and 8)

a) 6-[[3-Acetoxy-2-(diethoxyphosphorylmethoxy)propoxy]amino]-4-chloro-5-formamidopyrimidine

A mixture of 4,6-dichloro-5-formamidopyrimidine (0.77g, 4.0mmol), diethyl [2-acetoxy-1-(aminooxymethyl)ethoxy]-methylphosphonate (1.2g, 4.0mmol) and triethylamine (0,82ml, 6mmol) in dry dioxan (20ml) was heated at 100°C for 2 hours. The reaction mixture was cooled, filtered, and the filtrate evaporated to leave an oil which was chromatographed on silica gel (eluant dichloromethane:methanol 97:3) and gave the title compound as a yellow oil (1.08g, 60%). $^1$H NMR: $\delta_H$ [$(CD_3)_2SO$] 1.23 (6H,t, J=7Hz, $(OCH_2C\underline{H}_3)_2$), 2.03 (3H,s, $COCH_3$), 3.85-4.35 (11H,m, $(OC\underline{H}_2CH_3)_2$ + $OC\underline{H}_2P$ + $OC\underline{H}_2C\underline{H}C\underline{H}_2O$). m/z: observed 454.1020; $C_{15}H_{24}N_4O_8PCl$ ($M^+$) requires 454.0996.

b) 9-[3-Acetoxy-2-(diethoxyphosphorylmethoxy)propoxy]-6-chloropurine

A solution of 6-[[3-acetoxy-2-(diethoxyphosphorylmethoxy)propoxy]amino]-4-chloro-5-formamidopyrimidine (0.80g, 1.76mmol) in diethoxymethyl acetate (5ml) was heated at 120°C for 2 hours. After cooling to ambient temperature, the excess solvent was evaporated under reduced pressure. The residue obtained was dissolved in methanol (5ml) and .880 ammonia (1ml) and left at ambient temperature for 10 minutes. The solvent was removed under reduced pressure and the residue chromatographed on silica gel (eluant dichloromethane: methanol 98:2) to give the title compound as a yellow oil (0.68g, 88%). IR: $\nu_{max}$ (film) 3080, 3050, 2990, 2900, 1740, 1640, 1590, 1565, 1435, 1390, 1370, 1330, 1240, 1160, 1120, 1040, 1025, 970, 930, 850, 820, 780cm$^{-1}$. $^1$H NMR: $\delta_H$ [$(CD_3)_2SO$] 1.23 (6H,t, J=7Hz, $(OCH_2C\underline{H})_3)_2$), 2.04 (3H,s. $COCH_3$), 3.95-4.15 (7H,m, ($OCH_2P$ + $(OC\underline{H}_2CH_3)_2$ + CH), 4.20 (1H,dd, J=5.22Hz, J=12.1Hz, $C\underline{H}_BOAc$), 4.36 (1H,dd, J=3.85Hz, J=12.1Hz, $C\underline{H}_AOAc$), 4.55 (1H,dd, J=6.05Hz, J=11.55Hz, N-$OC\underline{H}_B$), 4.70 (1H,dd,J=3.58Hz, J=11.55Hz, N-$OCH_A$), 8.83 (1H,s), 9.04(1H,s). Found: C,41.38; H,5.13; N,12.39%; $C_{15}H_{22}N_4O_7PCl$ requires: C,41.24; H,5.08; N,12.83%. m/z: (FAB, thioglycerol matrix) 437 ($MH^+$).

Description 9 (Intermediates for Examples 16)

a) 6-[[3-Acetoxy-2-(diethoxyphosphorylmethoxy)propoxy]amino]-4-chloro-2,5-diformamidopyrimidine

A mixture of 4,6-dichloro-2,5-diformamidopyrimidine (0.475mg, 2mmol), diethyl [2-acetoxy-1-(aminooxymethyl)ethoxy)methylphosphonate (0.60mg, 2mmol) and triethylamine (0.54ml, 4mmol) in dry dioxan (20ml) was heated at 120 °C for 5 hours. The reaction mixture was cooled to ambient temperature, filtered, and the filtrate evaporated to leave a yellow oil. The oil was chromatographed on silica gel (eluant dichloromethane: methanol 97:3) to give the title compound as a pale yellow solid (0.81g, 80%). $^1$H NMR: $\delta_H$ [$(CD_3)_2SO$] 1.23 (2 x 3H, 2 x t, J = 7Hz, ($OCH_2C\underline{H}_3)_2$), 2.03 (3H, s, $COCH_3$), 3.80-4.35 (11H,m, $C\underline{H}_2C\underline{H}C\underline{H}_2$ + $(OC\underline{H}_2CH_3)_2$ + $OC\underline{H}_2P$), 8.16 (1H,br.s, sharpens to s. on $D_2O$, $C\underline{H}O$), 9.26 (1H,d,J = 9.9Hz, collapses to s. on $D_2O$, $C\underline{H}O$), 9.45 (1H,br.s, $D_2O$ exchangeable, NH), 10.8-10.95 (2H,m,$D_2O$ exchangeable 2 x NH). Found: C,37.36; H,5.37; N,13.43%; $C_{16}H_{25}N_5O_9PCl.H_2O$ requires: C,37.18; H,5.27; N,13.55%. m/z: observed 497.1082; $C_{16}H_{25}N_5O_9PCl(M^+)$ requires 497.1078.

b) 9-[3-Acetoxy-2-(diethoxyphosphorylmethoxy)propoxyl-6-chloro-2-formamidopurine

A solution of 6-[[3-acetoxy-2-(diethoxyphosphorylmethoxy)propoxy]amino]-4-chloro-2,5-formamidopyrimidine (0.30g, 0.66mmol) in diethoxymethyl acetate (5ml) was heated at 120 °C for 3 hours. After cooling to ambient temperature, excess solvent was removed under reduced pressure. The residue obtained was dissolved in methanol (2ml) and .880 ammonia (0.5ml) and left at ambient temperature for 15 minutes. The solvent was removed under reduced pressure and the residue obtained chromatographed on silica gel (eluant dichloromethane: methanol 97:3) to give the title compound as a colourless gum (0.20g, 63%). $^1$H NMR: $\delta_H$ [$(CD_3)_2SO$] 1.22 (2 x 3H, 2 x t,J = 7Hz, $(OCH_2C\underline{H}_3)_2$), 2.04 (3H, s, $COCH_3$), 3.75-4.20 (7H,m, $(OC\underline{H}_2CH_3)_2$ + $OC\underline{H}_2P$, $C\underline{H}$), 4.21 (1H,dd, J = 5.22Hz, J = 12.10Hz, $CH_BOAc$) 4.38 (1H,dd, J = 3.85Hz, J = 12.10Hz, $C\underline{H}_AOAc$), 4.50 (1H,dd, J = 6.05Hz, J = 11.55Hz, $NOCH_B$), 4.63 (1H,dd, J = 3.57Hz, J = 11.55Hz,$NOC\underline{H}_A$), 8.82 (1H,s, H-8), 9.38 (1H,d, J = 9.6 Hz, collapses to s. on $D_2O$, $C\underline{H}O$), 11.32 (1H,d,J = 9.6Hz, $D_2O$ exchangeable NH).

Description 10 (Intermediates for Examples 6 (Method A), 7 and 8)

a) 9-Benzyloxy-6-chloropurine

A mixture of 4,6-dichloro-5-formamidopyrimidine (58.6g; 0.31mmol), benzyloxyamine (37.5g; 0.31mmol), triethylamine (100ml) and dioxan (400ml) was stirred at 100 °C for 4 hours. The reaction was cooled, filtered and evaporated under reduced pressure. The residue was partitioned between ethyl acetate (750ml), saturated aqueous potassium bicarbonate (400ml) and brine (200ml). The organic phase was separated and the aqueous phase washed with ethyl acetate (300ml). The combined organic phases were washed with water (200ml), brine (200ml), dried ($MgSO_4$), and evaporated under reduced pressure.

The residue was dissolved in anhydrous N,N-dimethylformamide (100ml), triethyl orthoformate (200ml), and 12N hydrochloric acid (5ml). After 4 hours at 25 °C the solvent was removed under reduced pressure. The residue was partitioned between chloroform (750ml) and saturated aqueous potassium bicarbonate (500ml). The resulting suspension was filtered and the phases separated. The organic phase was washed with saturated potassium bicarbonate (200ml), water (200ml), dried ($MgSO_4$) and evaporated under reduced pressure. The residue was purified by column chromatography on silica gel eluting with chloroform-methanol (100:1), affording the title compound (38.4g, 48%). IR: $\nu_{max}$ (KBr) 3350, 1587, 1565, 1438 cm$^{-1}$; $^1$H NMR: $\delta_H$ ($CDCl_3$) 5.40(2H, s, $C\underline{H}_2Ar$), 7.35(5H, s, Ar), 7.75(1H, s, H-8), 9.85(1H, s, H-2). Found: C, 55.11; H, 3.73; N, 21.27%. $C_{12}H_9N_4OCl$ requires: C, 55.28; H, 3.49, N, 21.50%.

b) 9-Benzyloxyadenine

A solution of 9-benzyloxy-6-chloropurine (38.4g; 0.147mmol) in ethanol (300ml) saturated with ammonia was heated at 100 °C in an autoclave for 16 hours. After cooling the suspension was evaporated to dryness and the residue partitioned between chloroform (750ml) and water (500ml). The separated aqueous phase was washed with chloroform (200ml). The combined organic phases were washed with water, dried ($MgSO_4$) and evaporated, affording an orange solid homogeneous on t.l.c. (31.1g, 87%). IR $\nu_{max}$ (KBr) 3372, 3300, 3187, 3038, 1660, 1637, 1600, 1581 cm$^{-1}$; $^1$H NMR: $\delta_H$ [$(CD_3)_2SO$] 5.3(2H, s, $C\underline{H}_2Ar$), 6.8(2H, br.s, $D_2O$ exchangeable, $NH_2$), 7.3(6H, s, H-8, At), 7.7(1H, s, H-2). Found: C, 59.39; H, 4.60; N, 29.07%; m/e

241.0949. $C_{12}H_{11}N_5O$ requires: C, 59.73; H, 4.60; N, 29.03% m/z 241.0964.

c) 9-Benzyloxy-6-phthalimidopurine

Phthaloyl dichloride (13.35g; 92.2mmol) was added to a cooled solution of 9-benzyloxyadenine (14.9g, 61.5mmol), 4-dimethylaminopyridine (1.5g, 12.3mmol) and triethylamine (25.7ml, 184.4mmol) in tetrahydrofuran (200ml). After 1 hour at room temperature the solvent was removed under reduced pressure and the residue partitioned between chloroform (500ml) and saturated potassium bicarbonate (300ml). The organic phase was separated washed with water (200ml), brine (200ml), dried ($MgSO_4$) and evaporated to dryness. Column chromatography on silica gel eluting with chloroform-methanol (100:1) afforded the title compound (11.20g, 49%). IR: $\nu_{max}$ (KBr) 3070, 1800, 1740, 1730, 1605, 1585, 1450, and 1410 $cm^{-1}$; $^1H$ NMR $\delta_H$ [$(CD_3)_2SO$] 5.70(2H, s, $\underline{CH_2}Ph$), 7.55(5H, s, $CH_2\underline{Ph}$), 8.20 (4H, s, Ar), 8.95(1H, s, H-2), 9.25(1H, s, H-8). Found: C, 64.71, H, 3.78; N, 18.85%; m/z 371.1025. $C_{20}H_{13}H_5O_3$ requires: C, 64.68; H, 3.54; N, 18.86%; m/e 371.1018.

d) 9-Hydroxy-6-phthalimidopurine

A mixture of 9-benzyloxy-6-phthalimidopurine (11.0g, 29.5mmol), 10% palladium on charcoal (2.2g), ethanol (300ml) and tetrahydrofuran (500ml) was stirred at 25°C for 1 hour under an atmosphere of hydrogen. The suspension was then filtered and the catalyst washed with ethanol. The filtrate was evaporated under reduced pressure and the resulting solid triturated with ether. The solid was collected and then dried to afford the title compound (6.93g; 83%). IR: $\nu_{max}$ (KBr). 2607, 1794, 1735, 1603, 1582, 1467, 1401 $cm^{-1}$; $^1H$ NMR $\delta_H$ [$(CD_3)_2SO$] 8.15(4H, s, Ar), 8.95(1H, s, H-2), 9.15(1H, s, H-8), 12.80(1H, br.s, $D_2O$ exchangeable, OH). Found C, 55.34; H, 2.58; N, 24.56%. $C_{13}H_7N_5O_3$ requires: C, 55.51; H, 2.51; N, 24.91%.

e) 9-[3-Acetoxy-2-(diethoxyphosphorylmethoxy)propoxy]-6-N-phthalimidopurine

Diethyl azodicarboxylate (0.4ml, 2.6mmol) was added to a mixture of 9-hydroxy-6-N-phthalimidopurine (0.61g, 2.17mmol), diethyl [2-acetoxy-1-(hydroxymethyl)ethoxy]methylphosphonate (0.61g, 2.17mmol) triphenylphosphine (0.68g, 2.6mmol) in dry THF (20ml) at 0°C. A pale yellow solution was obtained within a few minutes and the reaction mixture was then stirred at ambient temperature for 2 hours. The solvent was evaporated under reduced pressure and the residue obtained chromatographed on silica gel using hexane: acetone 3:1 as eluant, before changing to acetone: hexane 3:1 to give the title compound as a colourless oil (0.95g, 80%). IR: $\nu_{max}$ (film) 3100, 3060, 2980, 2910, 1790, 1740, 1730, 1600, 1580, 1465, 1445, 1405, 1370, 1330, 1240, 1160, 1135, 1020, 980, 880, 790, 775$cm^{-1}$. $^1H$ NMR: $\delta_H$ [$(CD_3)_2SO$] 1.23 (2 x 3H, 2 x t, J=7Hz, $(OCH_2\underline{CH_3})_2$), 2.05 (3H,s, $COCH_3$), 3.95-4.45 (9H,m, $O\underline{CH_2}CH_3)_2$ + $O\underline{CH_2}P$, $\underline{CH}\underline{CH_2}OAc$), 4.55-4.80 (2H, m, $N\text{-}O\underline{CH_2}$), 8.0-8.20 (4H,m, aromatic H), 9.04 (1H,s) 9.11(1H,s). m/z: (FAB, thioglycerol matrix) 548 ($MH^+$, 100%).

f) 9-[3-Acetoxy-2-(diethoxyphosphorylmethoxy)propoxy]adenine

To a solution of 9-[3-acetoxy-2-(diethoxyphosphorylmethoxy)propoxy]-6-N-phthalimidopurine (0.91g, 1.66mmol) in dry dichloromethane (5ml) was added methylhydrazine (0.09ml, 1.1 eq) and the solution stirred at ambient temperature for 15 minutes. The reaction mixture was filtered, the white solid washed with dichloromethane and the combined filtrates evaporated to dryness. The residue obtained was chromatographed on silica gel (dichloromethane:methanol 95:5 as eluant) to give the title compound as a colourless oil (0.58g, 83%). IR: $\nu_{max}$ (film) 3330, 3190, 2990, 2900, 1740, 1660, 1640, 1595, 1470, 1410, 1370, 1330, 1295, 1240, 1050, 1020, 975, 820, 790$cm^{-1}$. $^1H$ NMR: $\delta_H$ [$(CD_3)_2SO$] 1.23 (2 x 3H, 2 x t, J=7Hz, $(OCH_2\underline{CH_3})_2$), 2.03 (3H,s, $COCH_3$), 4.0-4.15 (7H,m, $(O\underline{CH_2}CH_3)_2$ + $\underline{CH}$ + $O\underline{CH_2}P$), 4.15-4.37 (2H, 2xdd, J=12.10, 5.2, 4.1$H_z$, $\underline{CH_2}OAc$), 4.44 (1H,dd, J=11.3 $H_z$,J=6.0$H_z$,$NOC\underline{H_B}$), 4.55 (1H, dd, J=11.27Hz, J=3.85Hz, $NOC\underline{H_A}$), 7.38 (2H,br.s,$D_2O$ exchangeable $NH_2$) 8.15 (1H,s), 8.43(1H,s). Found: C,42.01; H,5.86; N,16.14%; $C_{15}H_{24}N_5O_7P$. $0.5H_2O$. requires: C,42.25; H,5.91; N,16.42%. m/z: observed 417.1412; $C_{15}H_{24}N_5O_7P$ requires: 417.1412.

Description 11 (Intermediates for Examples 9 Method B), 10 and 15)

a) 6-Benzyloxyamino-4-chloro-2,5-diformamidopyrimidine

A mixture of 4,6-dichloro-2,5-diformamido-pyrimidine (1.9g, 8.09mmol), benzyloxyamine (1g, 8.13mmol), triethylamine (2ml) and dioxan (20ml) was stirred at 100°C for 1 hour. The cooled reaction mixture was filtered and the precipitate collected and washed with dioxan (2 x 5ml). The filtrate and washings were combined and evaporated to a syrup. Column chromatography on silica gel (eluted with chloroform-ethanol, 30:1) afforded the title compound (1.2g, 46%). IR: $\nu_{max}$ (KBr) 3242, 1694, 1588, 1472cm$^{-1}$; $^1$H NMR $\delta_H$ [$(CD_3)_2SO$], 4.89 (2H, s, $OC\underline{H}_2Ph$), 7.4 (5H, m, Ph), 8.15 (1H, s, CHO), 9.18, 9.42 (1H, 2 x br.s, $D_2O$ exchangeable, NH), 9.25 (1H, br.s, CHO), 10.91 (2H, br.s, $D_2O$ exchangeable, 2 x NH). m/z (FAB +ve ion, thioglycerol) $MH^+$ 322.

b) 9-Benzyloxy-6-chloro-2-formamidopurine

6-Benzyloxyamino-4-chloro-2,5-diformamidopyrimidine (1.2g, 3.73mmol) and diethoxymethyl acetate (20ml) was stirred at 120°C for 2.5 hours, cooled and evaporated under reduced pressure. A solution of the residue in methanol (20ml) and .880 ammonia (2ml) was stirred at 20°C for 1 hour, the solvent removed under reduced pressure and the residue co-evaporated with methanol. Column chromatography on silica gel (eluted with chloroform ethanol, 100:1) afforded the title compound (700mg, 62%). IR: $\nu_{max}$ (KBr) 3119, 1702, 1611, 1577, 1505, 1440cm$^{-1}$; $^1$H NMR: $\delta_H$ [$(CD_3)_2SO$], 5.44 (2H, s, $C\underline{H}_2Ph$), 7.45 (5H, m, Ph), 8.54 (1H, s, H-8), 9.34 (1H, s, CHO), 11.30 (1H, br.s, $D_2O$ exchangeable, NH). Found: C, 49.99; H, 3.37; N, 22.43%, m/z 303.0523. $C_{13}H_{10}N_5O_2Cl$ + 0.5 $H_2O$ requires: C, 49.92; H, 3.55; N, 22.40%, m/z 303.0520.

c) 2-Amino-9-benzyloxy-6-methoxypurine

A mixture of 9-benzyloxy-6-chloro-2-formamidopurine (440mg, 1.60mmol), 1.2M sodium methoxide in methanol (5.3ml) and methanol (10ml) was heated at reflux temperature for 1 hour and then cooled. Acetic acid (4ml) was added and the solution evaporated to dryness. The residue was suspended in water and extracted with chloroform (2 x 25ml). The combined chloroform extracts were washed with brine, dried (magnesium sulphate) and evaporated under reduced pressure. Column chromatography on silica gel (eluted with chloroform-methanol, 100:1) afforded the title compound (331mg, 76%). IR: $\nu_{max}$ (KBr) 3480, 3310, 1625, 1585, 1505, 1485, 1460, 1400cm$^{-1}$; $^1$H NMR: $\delta_H$ [$(CD_3)_2SO$] 3.96 (3H, s, $CH_3$), 5.31 (2H, s, $C\underline{H}_2Ph$), 6.64 (2H, br.s, $D_2O$ exchangeable, $NH_2$), 7.42 (5H, s, Ph), 7.75 (1H, s, H-8). Found: C, 57.18, H, 4.84; N, 25.85%). m/z 271.1075. $C_{13}H_{13}N_5O_2$ requires: C, 57.56, H, 4.83, H, 25.82%; m/z 271.1069.

d) 9-Benzyloxy-2-[(bis-t-butoxycarbonyl)amino]-6-methoxypurine

A solution of 2-amino-9-benzyloxy-6-methoxypurine (0.47g; 1.73mmol), di-t-butyldicarbonate (0.57g; 2.60mmol) and 4-N,N-dimethylaminopyridine (100mg, 0.173mmol) in tetrahydrofuran was heated at reflux for 45 minutes. Additional di-t-butyldicarbonate (0.20g) was then added and the solution refluxed for 30 minutes. The reaction was then cooled and the solvent removed under reduced pressure. The residue was purified by column chormatography on silica gel eluting with chloroform-methanol mixtures, affording the title compound (740mg; 91%). IR: $\nu_{max}$ (KBr) 3110, 2990, 1760, 1600, 1485, 1460, 1400 cm$^{-1}$; $^1$H NMR: $\delta_H$ - ($CDCl_3$) 1.50(18H, s, 6 x $CH_3$), 4.15(3H, s, $CH_3$), 5.45(2H, s, $CH_2$), 7.35(5H, s, Ar), 7.65(1H, s, H-8).

e) 2-[(Bis-t-butoxycarbonyl)amino]-9-hydroxy-6-methoxypurine

A mixture of 9-benzyloxy-2-[(bis-t-butoxycarbonyl)amino]-6-methoxypurine (990mg; 2.10mmol), 10% palladium on charcoal (100mg), ethanol (25ml) and dioxan (25ml) was stirred at 20°C under an atmosphere of hydrogen for 45 minutes. The suspension was then filtered and the filtrate evaporated under reduced pressure. The resulting white solid was dried to yield the title compound (760mg; 95%). IR: $\nu_{max}$ (KBr) 2990, 2420, 1760, 1740, 1730, 1710, 1605, 1480 cm$^{-1}$; $^1$H NMR: $\delta_H$ [$(CD_3)_2SO$] 1.40 (18H, s, 6 x $CH_3$), 4.05(3H, s, $OCH_3$), 8.05(1H, s, H-8), 11.8(1H, br.s, $D_2O$ exchangeable, OH). Found: C, 50.27; H, 6.12; N, 17.70% $C_{16}H_{23}N_5O_6$ + 0.2EtOH requires: C, 50.42; H, 6.23; N, 17.66%.

f) 9-[3-Acetoxy-2-(diethoxyphosphorylmethoxy)propoxy]2-[bis-(t-butoxycarbonyl)amino]-6-methoxypurine

Diethyl azodicarboxylate (0.49ml, 3.15mmol) was added to a mixture of 2-[bis-(t-butoxycarbonyl)amino]-9-hydroxy-6-methoxypurine (1g, 2.6mmol) triphenylphosphine (0.82g, 3.15mmol) and diethyl [2-acetoxy-1-(hydroxymethyl)ethoxy]methylphosphonate (0.74g, 2.6mmol) in dry THF (20ml) at 0 ° C. The reaction mixture was stirred at ambient temperature for 3 hours, the solvent removed under reduced pressure and the residue obtained chromatographed on silica gel (haxane:acetone 3:1 as eluant, then hexane:acetone 1:1 and finally hexane:facetone 1:3) to give the title compound as a colourless oil (1g, 59%). IR: $\nu_{max}$ (film) 2980, 2940, 1790, 1740, 1595, 1490, 1425, 1370, 1280, 1250, 1165, 1120, 1100, 1050, 970, 850, 790cm$^{-1}$. $^1$H NMR: $\delta_H$ [$(CD_3)_2SO$] 1.22 (2 x 3H, 2 x t, J = 7Hz, ($OCH_2C\underline{H}_3)_2$), 1.41 (18H,s, 2 x $C(CH_3)_3$), 2.02 (3H,s, $COCH_3$), 3.75-4.40 (9H,m, ($OC\underline{H}_2CH_3)_2$ + $OCH_2P$ + $C\underline{H}C\underline{H}_2OAc$), 4.08 (3H,s,$OCH_3$), 4.48 (1H, dd, J = 6.05Hz, J = 11.27Hz, $NOC\underline{H}_B$), 4.58 (1H,dd,J = 3.3Hz, J = 11.27Hz, $NOC\underline{H}_A$), 8.76 (1H,s,H-8). m/z: (FAB, thioglycerol matrix) 648 ($MH^+$, 9%).

Example 1

9-[2-(Diethoxyphosphorylmethoxy)ethoxy]adenine

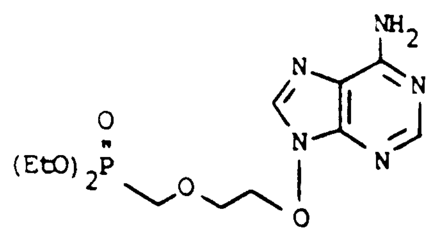

A solution of 6-chloro-9-[2-(diethoxyphosphorylmethoxy)ethoxy]purine (1.45g, 4mmol) in ethanol (20ml) saturated with ammonia gas was heated in a sealed steel vessel at 100 ° C for 2 hours. The reaction mixture was cooled to ambient temperature, evaporated to dryness and the residual oil chromatographed on silica gel (eluted with dichloromethane: methanol, 90:10) to give recovered starting material (0.25g) and the title compound (0.94g, 82% - based on used starting material), crystallised from acetone-ether, m.p. 74-76 °. UV:$\lambda_{max}$ (EtOH) 260nm ($\epsilon$ 13,500); IR: $\nu_{max}$ (KBr) 3308, 3146, 1669, 1646, 1600, 1538, 1466, 1416, 1320, 1301, 1232, 1206, 1164, 1129, 1066, 1053, 1039, 1009, 978 cm$^{-1}$; $^1$H NMR: $\delta_H$ [$(CD_3)_2SO$] 1.24 (6H,t,J = 7Hz, ($OCH_2C\underline{H}_3)_2$), 3.83-3.89 (2H,m,$C\underline{H}_2OCH_2P$), 3.91 (2H,d,J = 8Hz, $OC\underline{H}_2P$), 4.0-4.15 (4H, dq, J = 7Hz, 7Hz, ($OC\underline{H}_2CH_3)_2$), 4.48-4.51 (2H,m,N-$OC\underline{H}_2$), 7.37 (2H,br.s,$D_2O$ exchangeable $N\underline{H}_2$) 8.14 (1H,s,H-8), 8.34 (1H,s,H-2). Found : C,41.74; H: 5.49; N: 20.06%; m/z 345.1202. $C_{12}H_{20}N_5O_5P$ requires: C,41.74; H, 5.83; N, 20.28%; m/z 345.1212.

Example 2

9-[2-(Phosphonemethoxy)ethoxy]adenine

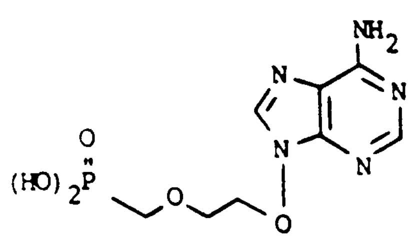

To a solution of 9-[2-(diethoxyphosphorylmethoxy)ethoxy]adenine (0.63g, 1.83mmol) in dry dichloromethane (10ml) was added trimethylsilyl bromide (1.45ml, 11mmol) and the solution stirred at ambient temperature

for 2 hours. The solvent was removed under reduced pressure and the residue dissolved in methanol (10ml). The solvent was evaporated and the residue again dissolved in methanol and solvent evaporated to give a solid residue. Crystallisation from methanol: acetone gave the title compound which was recrystallised from methanol: water (0.24g, 45%), m.p. 241-244 °C.
UV:$\lambda_{max}$ (EtOH) 259nm ($\epsilon$ 13,500); IR: $\nu_{max}$ (KBr) 3428, 3312, 3084, 1685, 1644, 1612, 1484, 1462, 1455, 1336, 1294, 1278, 1255, 1215, 1195, 1104, 1060, 1046, 1033, 955, 934, 892, $cm^{-1}$. $^1H$ NMR: $\delta_H$ [$(CD_3)_2SO$] 3.64 (2H,d,J = 8.2Hz, $OC\underline{H}_2P$), 3.82 (2H,m,N-$OCH_2C\underline{H}_2O$), 4.50 (2H,m, N-$OC\underline{H}_2CH_2$), 7.42 (2H, br.s, $D_2O$ exchangeable $N\underline{H}_2$), 8.15 (1H,s,H-8), 8.38 (1H,s,H-2) 10.0-4.5 (2H,broad, $D_2O$ exchangeable $PO(O\underline{H})_2$).
Found: C,32.84; H,4.41; N: 23.74%. $C_8H_{12}N_5O_5P$. $H_2O$
requires: C,33.01; H,4.19; N:24.06%.

Example 3

9-[2-(Diethoxyphosphorylmethoxy)ethoxy]guanine

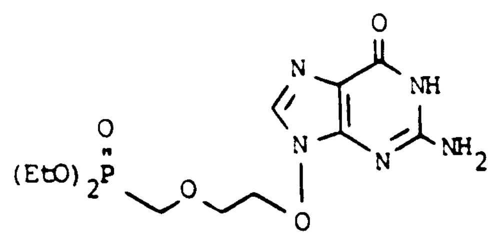

A solution of 6-chloro-9-[2-(diethoxyphosphorylmethoxy)ethoxy]-2-formamidopurine (2.1g, 5.1mmol) in 80% aqueous formic acid (15ml) was stirred and heated at 80 °C for 1.5 hours. After cooling to ambient temperature, the reaction mixture was evaporated to dryness under reduced pressure, the residue dissolved in methanol (20ml) and .880 ammonia (2ml) and left at ambient temperature for 1 hour. The solvent was evaporated and the residue obtained chromatographed on silica gel (eluted with dichloromethane: methanol, 90:10) to give the title compound (1.5g, 80%), which was crystallised from ethanol (0.8g, 43%) m.p. 176-178 °. UV: $\lambda_{max}$ ($H_2O$) 253 ($\epsilon$ 13,300)nm; $\lambda_{sh}$ ($H_2O$) 275 ($\epsilon$ 9050)nm; IR: $\nu_{max}$ (KBr) 3334, 3156, 1693, 1646, 1601, 1590, 1242, 1163, 1054, 1025, 970 $cm^{-1}$; $^1H$ NMR: $\delta_H$ [$(CD_3)_2SO$] 1.24 (6H,t,J = 7Hz, $(OCH_2C\underline{H}_3)_2$), 3.80 (2H,m, N-$OCH_2C\underline{H}_2O$), 3,90 (2H,d,J = 8Hz, $OC\underline{H}_2P$), 4.06 (4H,dq,J = 7Hz,7Hz, $(OC\underline{H}_2CH_3)_2$), 4.39 (2H,m, N-$OC\underline{H}_2CH_2$), 6.60 (2H,br.s, $D_2O$ exchangeable, $N\underline{H}_2$), 7.89 (1H,s,H-8), 10.67 (1H,br.s, $D_2O$ exchangeable N-1(H)).
Found: C,39.45; H,5.47; N,19.05%. $C_{12}H_{20}N_5O_6P$
requires: C,39.89; H,5.58; N,19.39%.

Example 4

9-[2-(Phosphonomethoxy)ethoxy]guanine

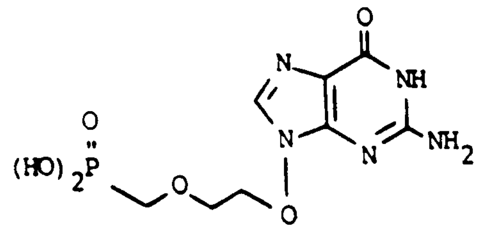

Method A

To a solution of 9-[2-(diethoxyphosphorylmethoxy)ethoxy]guanine (100mg, 0.28mmol) in dry dimethylformamide (2ml) was added trimethylsilyl bromide (0.22ml, 1.66mmol). The reaction mixture was stirred for 16 hours at ambient temperature, evaporated to dryness and the residue obtained dissolved in methanol (5ml). After 5 minutes at ambient temperature, the methanol was removed by evaporation. The residue was again treated wtih methanol as indicated above. The solid obtained was insoluble in water and methanol. This solid was suspended in water and .880 ammonia added until the solution pH reached 9-10. The basic solution was added to Dowex 50W-X8 resin (prewashed with 0.1M sodium hydroxide and then water until the washings were neutral) and the resin washed with water. Fractions containing UV absorbing material were combined and acidified wtih 2M HCl to precipitate a solid. The precipitate was collected and triturated with methanol to give the title compound as a white solid (50mg, 60%), m.p. 275-278 °C. UV: $\lambda_{max}$ (EtOH) 254nm ($\epsilon$ 10800); $\lambda_{sh}$ (EtOH) 278nm ($\epsilon$ 6400); IR: $\nu_{max}$ (KBr) 3378, 3161, 1705, 1649, 1602, 1459, 1382, $cm^{-1}$; $^1H$ NMR: $\delta_H$ [$(CD_3)_2SO$] 3.60 (2H,d,J=8.25Hz, $OC\underline{H}_2P$), 3.77 (2H,m,-$C\underline{H}_2OCH_2P$), 4.37 (2H,m, N-$O\underline{CH}_2CH_2$), 6.63(2H,br.s, $D_2O$ exchangeable $\underline{NH}_2$), 7.92 (1$\underline{H}$,s,H-8); 10.67 (1H,br.s,$D_2O$ exchangeable N$\underline{H}$), 2.6-5.5 (2H,board, phosphonic acid H).

Found: C,30.79; H,4.24; N,21.96%. $C_8H_{12}N_5O_6P$. $0.5H_2O$

requires: C,30.59; H,4.17; N,22.30%.

Method B

To a solution of 6-chloro-9-[2-(diethoxyphosphorylmethoxy)ethoxy]-2-formamidopurine (0.25g, 0.61mmol) in methanol (5ml), was added sodium methoxide solution (30 wt % in methanol; 0.5ml, 2.62mmol). The mixture was stirred and heated at 50 °C for 4 hours. After cooling to ambient temperature, 80% aqueous acetic acid (0.5ml) was added to give a solution of pH6. The solution was evaporated to dryness and the residue partitioned between dichloromethane (10ml) and aqueous sodium bicarbonate solution (10ml). The aqueous phase was washed with dichloromethane (2 x 10ml), the combined organic extracts dried (magnesium sulphate), and evaporated to dryness under reduced pressure. The residue obtained was chromatographed on silica gel (eluant dichloromethane: methanol 90:10) to give a 2-amino-6-methoxypurine derivative (0.20g) which was used without any further purification. The chromatographed material (0.20g) was dissolved in dichloromethane (2ml), trimethylsilyl bromide (0.7ml, 5.3mmol) added, and the solution left at ambient temperature for 2 days. The solvent was evaporated under reduced pressure and the residue obtained co-evaporated with methanol (x2) to leave a white solid which crystallised from hot water giving the title compound as a white solid (0.105g, 56%) m.p. 275-278 °C, identical in all respects with the compound obtained in Method A.

Example 5

9-[2-[Ethoxy(hydroxy)phosphorylmethoxy)ethoxy]guanine

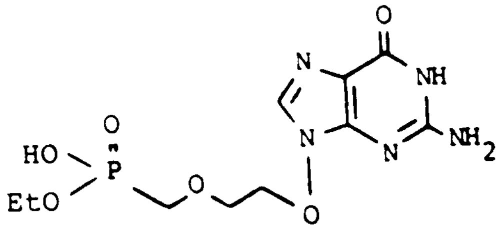

A solution of 9-[2-(diethoxyphosphorylmethoxy)ethoxy]guanine (100mg, 0.28mmol) in 10% (w/w) sodium hydroxide solution (3ml) was heated under reflux for 4 hours. After cooling to ambient temperature, the solution was neutralised with 12M HCl and evaporated to dryness to give a yellow solid. The solid was purified by chromatography on reverse phase silica eluting initially with water, then methanol-water (5:95) and finally methanol-water (10:90). UV absorbing fractions were collected and evaporated to dryness to leave the title compound as a white solid, m.p. 244-246° (25mg, 27%).$^1$H NMR: $\delta_H$ [$(CD_3)_2SO$ + $C_5D_5D$] 1.18 (3H, t, J=7Hz, $OCH_2CH_3$), 3.74 (2H, d, J=8.5 Hz, $OCH_2P$), 3.80-3.85 (2H, m,$CH_2OCH_2P$), 3.98 (2H, m, P-$OCH_2CH_3$), 4.40-4.45 (2H, m, N-$OCH_2$), 7.07 (2H, br.s, $D_2O$ exchangeable, $NH_2$), 8.01 (1H, s, H-8), 11.40 (1H, br.s, $D_2O$ exchangeable, NH), 4.1 - 4.7 (br.s, P-OH + water in solvent). Found: C, 35.34; H, 4.78; N, 20.40%. $C_{10}H_{16}N_5O_6P$. $0.5H_2O$ requires: C, 35.09; H, 5.00; N, 20.46%.

Example 6

9-[2-(Diethoxyphosphorylmethoxy)-3-hydroxypropoxy]adenine

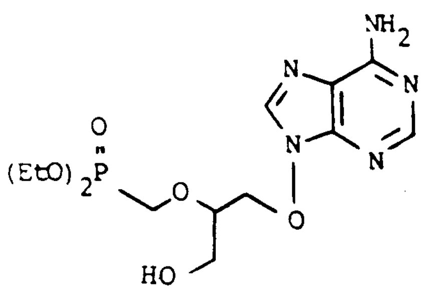

Method A

A solution of 9-[3-acetoxy-2-(diethoxyphosphorylmethoxy)propoxy]adenine (0.58g, 1.39mmol) in ethanol (10ml) and 2MHCl (1.5ml, 3.0mmol) was heated under reflux for 16 hours. The solution was cooled to ambient temperature and evaporated to dryness. The residue obtained was dissolved in ethanol and .880 ammonia solution added to give a final solution of pH 8.5. The solvent was removed under reduced pressure and the residue obtained chromatographed on silica gel (eluant dichloromethane:methanol 95:5, then 92:8, and finally 90:10) to give the title compound as a pale yellow oil (0.42g, 80%). $^1$H NMR: $\delta_H$ [-$(CH_3)_2SO$] 1.23 (6H,t,J=7Hz, $(OCH_2CH_3)_2$), 3.57 (2H,m, $CH_2OH$), 3.79 (1H,m,CH), 3.95-4.15 (6H,m, $OCH_2P$, $(OCH_2CH_3)_2$), 4.38 (1H,dd.J=6.6Hz, J=11.27Hz, $NOCH_B$), 4.54 (1H,dd,J=3.03Hz, J=11.27Hz, $NOCH_A$), 4.88 (1H,t, $D_2O$ exchangeable OH), 7.38 (2H,br.s,$D_2O$ exchangeable $NH_2$), 8.15 (1H,s), 8.43 (1H,s). Found: C,38.72; H,6.06; N,17.50%; $C_{13}H_{22}N_5O_6P.1.5H_2O$ requires: C,38.80; H,6.01; N,17.41%. m/z: observed: 375.1306; $C_{13}H_{22}N_5O_6P(M^+)$ requires:375.1308.

Method B

To a solution of 9-[3-benzyloxy-2-(diethoxyphosphorylmethoxy)propoxy]adenine (300mg, 0.65mmol) in 80% aqueous acetic acid (10ml) was added 10% Pd-C (30mg) and the mixture shaken under a hydrogen atmosphere at ambient temperature and pressure for a total of 8 hours. The solution was filtered and the filtrate evaporated. The residue obtained was chromatographed on silica (dichloromethane/methanol 95:5, then 90:10 as eluants) to give the title compound, (100mg, 40%).

Method C

A solution of 9-[3-acetoxy-2-(diethoxyphosphorylmethoxy)propoxy]-6-chloropurine (0.63g, 1.44mmol) in ethanolic ammonia (12ml) was heated in a sealed vessel at 100 °C for 5 hours. After cooling to ambient temperature, the solvent was removed under reduced pressure to leave a brown solid. The solid was chromatographed on silica gel (dichloromethane:methanol 90:10 as eluant) and separated into 9-[3-acetoxy-2-(diethoxyphosphorylmethoxy)propoxy]adenine (0.29g) and 9-[2-(diethoxyphosphorylmethoxy)-3-hydroxypropoxy] adenine. The acetate derivative was hydrolysed as in method A, and the product of the hydrolysis combined with the initially chromatographed alcohol to give the title compound as a pale yellow oil (0.30g, 55%).

Example 7

9-[3-Hydroxy-2-(phosphonomethoxy)propoxy]adenine and

Example 8

9-[2-[ethoxy(hydroxy)phosphorylmethoxy]-3-hydroxypropoxy]adenine

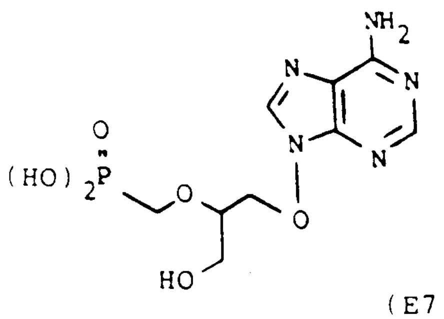

(E7)

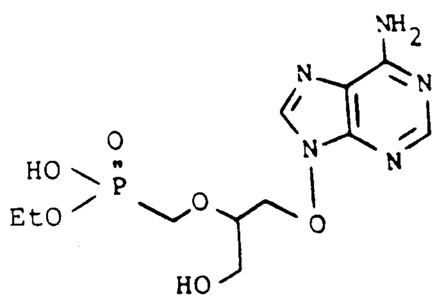

(E8)

To a solution of 9-[2-(diethoxyphosphorylmethoxy)-3-hydroxypropoxy]adenine (90mg, 0.24mmol) in dry dichloromethane (2.5ml) and dry DMF (0.5ml), was added bromotrimethylsilane (0.19ml, 1.44mmol). After 2 hours stirring at ambient temperature, the solvent was evaporated and the residue obtained dissolved in methanol. The solution was evaporated to dryness again and the residue again dissolved in methanol and evaporated. The residue was chromatographed on reverse phase silica [water (2 column volumes), then 5% methanol:water (3 column volumes), then 10% methanol: water (3 column volumes) as eluants] to give the diacid (17mg, 22%), mp 197-203 °, and the monoacid-monoester (30mg, 36%) mp 90-95 °, after lyophilisation.

Data for diacid, (Example 7). IR: $\nu$max (KBr disc) 3550-2100 (broad), 1704, 1608, 1467, 1418, 1340, 1304, 1229, 1207, 1163, 1125, 1079, 925, 889, 778, 757, 730 $cm^{-1}$. $^1H$ NMR: $\delta_H$ [$(CD_3)_2SO$] 3.54 (2H, m, $CHCH_2OH$), 3.75 (3H, m, $OCH_2P$ + $CH_2CHCH_2$), 4.3-4.56 (2H,m, N-$OCH_2$), 7.39 (2H, br.s, $D_2O$ exchangeable $NH_2$), 8.15 (1H, s), 8.48 (1H, s), 3.45 (3H, br.s, $D_2O$ exchangeable $PO(OH)_2$ + $CH_2OH$) UV: λ max (EtOH) 259nm. Found: C, 31.02; H, 4.50; N, 19.85%. $C_9H_{14}N_5O_5P$. $1.5H_2O$ requires: C, 31.22; H, 4.94; N, 20.23%. m/z: (FAB. thioglycerol matrix) 320 ($MH^+$).

Data for monoester, (Example 8). IR: $\nu$max (KBr) 3600-2200 (broad), 1692, 1647, 1602, 1472, 1413, 1333, 1296, 1166, 1128, 1047, 953, 882, 795, 781, 729. UV: λmax (EtOH) 259nm. $^1H$ NMR: $\delta_H$ [$(CD_3)_2SO$] 1.9 (3H, t, J = 7Hz, P-($OCH_2CH_3$), 3.54 (2H, m, $CHCH_2OH$), 3.74 (1H, m, $CHCH_2OH$) 3.83 (2H, d, J = 8.8Hz,

$OCH_2P$), 3.96 (2H, m, P-O$CH_2$$CH_3$), 4.3-4.55(2H, m, N-O$CH_2$), 7.39 (2H, br.s, $D_2O$ exchangeable $NH_2$), 8.15 (1H, s), 8.46 (1H, s), 3.4 (2H, br.s, $D_2O$ exchangeable P-OH + $CH_2OH$). Found: C, 37.10; H, 5.29; N, 19.59%. $C_{11}H_{18}N_5O_6P$. $0.5H_20$ requires: C, 37.08; H, 5.37; N, 19.65%. m/z: (FAB: thioglycerol matrix) 348 ($MH^+$).

Example 9

9-[2-(Diethoxyphosphorylmethoxy)-3-hydroxypropoxy]guanine

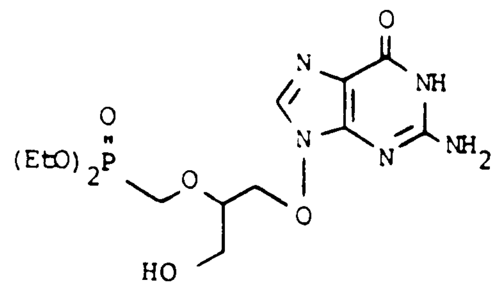

Method A

A mixture of 9-[3-benzyloxy-2-(diethoxyphosphorylmethoxy)propoxy]guanine (300mg, 0.6mmol) and 5% Pd-C (30mg) in 80% aqueous acetic acid (10ml) was shaken under a hydrogen atmosphere at ambient temperature and pressure until hydrogen uptake had ceased - a total of 16 hours. The hydrogenation mixture was filtered and the filtrate evaporated. The residue was chromatographed on silica gel (dichloromethane/methanol 85:15 as eluant) to give the title compound as a white solid (84mg, 30%) mp. 129-133°. IR:$\nu$max (KBr) 3335, 3150, 2984, 2933, 1693, 1648, 1602, 1476, 1382, 1244, 1164, 1116, 1053, 1026, 972 $cm^{-1}$. $^1H$ NMR: $\delta_H$ [$(CD_3)_2SO$] 1.23 (6H, t, J=7$H_z$, $(OCH_2CH_3)_2$), 3.45-3.65 (2H, m, $CH_2OH$), 3.7-3.85 (1H, m, $CH_2CHCH_2$), 3.9-4.2 (6H, m, $(OCH_2CH_3)_2$ + $OCH_2P$), 4.2-4.5 (2H, m, N-$OCH_2CH$), 4.84 (1H, t, J=5.5Hz, $D_2O$ exchangeable OH), 6.6 (2H, s, $D_2O$ exchangeable $NH_2$), 7.96 (1H, s, H-8), 10.69 (1H, br.s, $D_2O$ exchangeable NH). Found; C, 39.15; H, 5.50; N, 17.14%. $C_{13}H_{22}N_5O_7P$. $O.5H_2O$ requires C, 39.00; H, 5.79; N, 17.49%. m/z: (positive ion FAB) 392 ($M+H^+$).

Method B

A solution of 9-[3-acetoxy-2-(diethoxyphosphorylmethoxy)propoxy]-2-[bis-t-butoxycarbonyl)amino]-6-methoxypurine (0.28g) 0.43mmol) in ethanol (4ml) and 2MHCl (1ml) was heated under reflux for 5 hours. The solution was cooled and evaporated to dryness. The residue obtained was chromatographed on silica gel (pre-absorbed onto silica gel in ethanol; dichloromethane:methanol 80:20 as eluant) to afford 9-[2-(diethoxyphosphorylmethoxy)-3-hydroxypropoxy]guanine as a pale yellow solid (0.08g, 47%).

Example 10

9-[3-Hydroxy-2-(phosphonomethoxy)propoxy]guanine

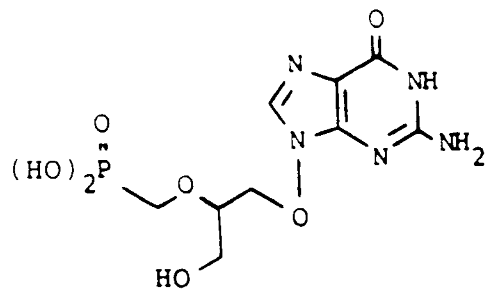

To a solution of 9-[2-(diethoxyphosphorylmethoxy)-3-hydroxypropoxy]guanine (60mg, 0.15mmol) in dry dimethylformamide (1ml) was added bromotrimethylsilane (0.5ml, 3.75mmol). The solution was kept at ambient temperature for 3 hours after which the solution was evaporated to dryness under reduced pressure. The residue obtained was dissolved in methanol and the solution evaporated to dryness again to leave an off-white solid. This solid was triturated with methanol to give the title compound as a white solid, m.p. >300°, (34mg, 66%). IR $\nu$max (KBr). 3385, 3159, 1715, 1645, 1598, 1384, 1157, 1109, 1060, 930, 772 $cm^{-1}$. $^1H$ NMR: $\delta_H$ $[(CD_3)_2SO]$, 3.52 (2H, m, $C\underline{H}_2OH$), 3.60-3.80 (3H, m, $OC\underline{H}_2P$ + $CH_2C\underline{H}$) 4.25 (1H, dd, J=11Hz, J=7.1Hz, N-$OC\underline{H}_B$), 4.40 (1H, dd, J=11Hz, J=3.3Hz N-$OC\underline{H}_A$), 6.61 (2H, br.s, $D_2O$ exchangeable $NH_2$), 8.00 (1H, s, H-8), 10.65 (1H, br.s, $D_2O$ exchangeable N$\underline{H}$) 2.6-4.1 (3H, broad, $D_2O$ exchangeable O$\underline{H}$, $PO(O\underline{H})_2$). Found: C, 31.59; H, 4.11; N, 20.42%. $C_9H_{14}N_5O_7P$. $0.25H_2O$ requires: C, 31.81, H, 4.27; N, 20.61%. m/z: (positive ion FAB) 336 $(M+H^+)$.

Example 11

2,6-Diamino-9-[2-(diethoxyphosphorylmethoxy)ethoxy]purine

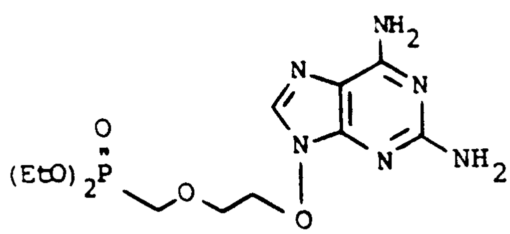

A solution of 6-chloro-9-[2-(diethoxyphosphorylmethoxy)ethoxy]-2-formamidopurine (0.80g, 1.96mmol) in ethanol (10ml) saturated with ammonia gas was heated in a sealed steel vessel at 110°C for $5\frac{1}{2}$ hours. The reaction mixture was cooled to ambient temperature, evaporated to dryness and the residue chromatographed on silica gel (eluted with dichloromethane:methanol 90:10) to give the title compound (0.25g, 35%), m.p. 139-141° (acetonitrile). UV: $\lambda_{max}$ (EtOH) 256 ($\epsilon$ 8,200), 280 ($\epsilon$ 10,200)nm:
IR $\nu_{max}$ (KBr) 3443, 3404, 3320, 3152, 2984, 2940, 1662, 1644, 1608, 1586, 1505, 1480, 1414, 1403, 1360, 1338, 1277, 1265, 1241, 1229, 1221, 1187, 1163, 1113, 1098, 1052, 1024, 978, 881, 842, 789, 769, 750 $cm^{-1}$. $^1H$ NMR: $\delta_H[(CD_3)_2SO]$ 1.24 (6H, t, J=7Hz, $(OCH_2C\underline{H}_3)_2$), 3.81 (2H, m, $C\underline{H}_2$), 3.92 (2H, d, J=7.98Hz, $OC\underline{H}_2P$), 4.06 (4H, m, $(OC\underline{H}_2CH_3)_2$), 4.38 (2H, m, N-$OC\underline{H}_2$), 5.93 (2H, br.s, $D_2O$ exchangeable $NH_2$), 6.79 (2H, br.s, $D_2O$ exchangeable $NH_2$), 7.88 (1H, s, H-8).
Found: C,39.57; H,5.82; N,23.27%; $C_{12}H_{21}N_6O_5P$. $0.1H_2O$
requires C,39.80; H,5.90; N,23.21%. m/z: observed 360.1318; $C_{12}H_{21}N_6O_5P(M^+)$ requires 360.1312.

Example 12

2,6-Diamino-9-[2-phosphonomethoxy)ethoxy]purine

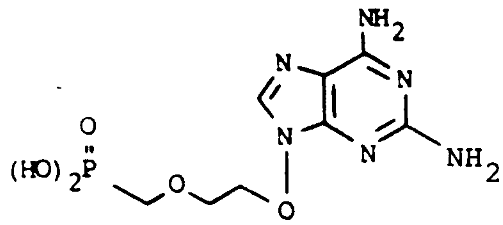

To a solution of 2,6-diamino-9-[2-(diethoxyphosphorylmethoxy)ethoxy]purine (0.15g, 0.41mmol) in dry dichloromethane (3ml) and dry dimethylformamide (1ml) was added trimethylsilyl bromide (0.55ml, 4.1mmol) and the solution stirred at ambient temperature for 2 hours. The solvent was removed under reduced pressure and the residue dissolved in methanol. The solution was evaporated to dryness and the solid residue obtained triturated with methanol to give the title compound (75mg, 59%) m.p. >300°. IR: $\nu_{max}$ (KBr) 3369, 3145, 3138, 3120, 1658, 1617, 1593, 1532, 1485, 1455, 1419, 1405, 1368, 1285, 1232, 1163, 1118, 1059, 915, 886, 841, 781, 766, 741, 716cm$^{-1}$. $^1$H NMR: $\delta_H$ [$(CD_3)_2SO$] 3.60 (2H, d, J=8.25Hz, O$\underline{CH_2}$P), 3.75 (2H, m, $\underline{CH_2}$), 4.37 (2H, m, NO$\underline{CH_2}$), 6.0 (2H, br.s, $D_2O$ exchangeable $NH_2$), 6.82 (2H, br.s, $D_2O$ exchangeable $NH_2$), 7.93 (1H, s, H-8), 2.5-5.0 (broad, $D_2O$ exchangeable PO(O$\underline{H}$)$_2$ + water from solvent). Found: C,30.28; H,4.81; N,26.03%. $C_8H_{13}N_6O_5P$. $0.9H_2O$ requires; C,29.99; H,4.65; N,26.23%.

Example 13

2-Amino-6-chloro-9-[2-(phosphonomethoxy)ethoxy]purine

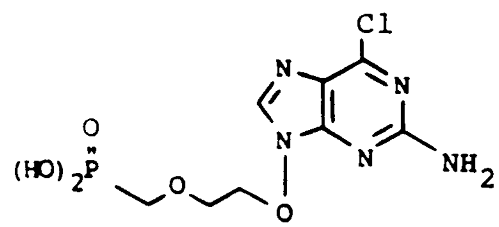

To a solution of 6-chloro-9-[2-diethoxyphosphorylmethoxy)ethoxy]-2-formamidopurine (0.370g, 0.91mmol) in dry dimethylformamide (5ml) was added trimethylsilyl bromide (1.3ml, 9.6mmol) and the solution stirred at ambient temperature for 3 hours. The solvent was removed under reduced pressure and the residue co-evaporated with acetone:water 1:1 (x 2). The solid residue obtained was crystallised from hot water to give the title compound as a tan solid (0.15g, 51%), m.p. 190-195°C. IR:$\nu_{max}$ (KBr) 3480, 3430, 3200, 3150, 2920, 1660, 1625, 1570, 1525, 1480, 1370, 1320, 1235, 1195, 1125, 1105, 1030, 995, 950, 920, 880, 780cm$^{-1}$. $^1$H NMR: $\delta_H$ [$(CD_3)_2SO$] 3.63 (2H, d, J=8.52Hz, O$\underline{CH_2}$P), 3.81 (2H, m, $\underline{CH_2}OCH_2P$), 4.45 (2H, m, N-O$\underline{CH_2}$), 2.75-5.25 (2H, broad, $D_2O$ exchangeable $PO(OH)_2$), 7.11 (2H, br.s, $D_2O$ exchangeable $NH_2$), 8.37 (1H, s, H-8). Found: C,28.89; H,3.51; N,21.15%. $C_8H_{11}N_5O_5PCl$. $0.5H_2O$ requires: C,28.88; H,3.63; N,21.06%.
m/z: (FAB, thioglycerol matrix) 324 ($MH^+$, 100%).

Example 14

2-Amino-6-methoxy-9-[2-(phosphonomethoxy)ethoxy]purine

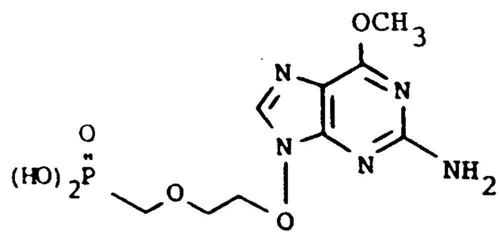

To a solution of 6-chloro-9-[2-(phosphonomethoxy)-ethoxy]guanine (0.10g, 0.31mmol) in methanol (3ml), was added sodium methoxide (0.59ml of 30 wt% solution in methanol, 3.1mmol) and the reaction mixture stirred at 50°C for 5 hours. The solution was cooled to ambient temperature, diluted with methanol (20ml) and Amberlite™ IR-120(plus) resin added. (A small excess of the resin was added to obtain a solution of pH 2.5). The solution was filtered and the resin washed with methanol:water (1:1, 30ml) and the combined filtrates concentrated under reduced pressure. The residue was purified by reverse-phase silica column chromatography using water as eluant to give the title compound as a white solid (70mg, 71%). UV: $\nu_{max}$ - ($H_2O$) 248 ($\epsilon$ 7,230), 280 ($\epsilon$ 8250)nm. $^1H$ NMR: $\delta_H$ [$(CD_3)_2SO$] 3.38 (2H, d, J=8.35Hz, $OCH_2P$), 3.70 (2H, m, $CH_2$), 3.95 (3H, s, $OCH_3$), 4.37 (2H, m, $NOCH_2$), 4.4-5.0 (2H, br.s, $D_2O$ exchangeable $PO(OH)_2$), 6.73 (2H, br.s, $D_2O$ exchangeable $NH_2$), 8.19 (1H, s, H-8).

Example 15

9-[(2-Hydroxy-2-oxo-1,4,2-dioxaphosphorinan-5-yl)-methoxy]guanine

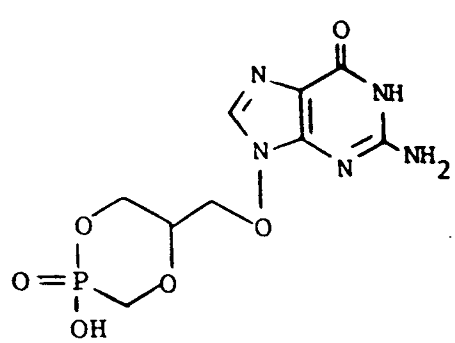

A solution of dicyclohexylcarbodiimide (0.323g, 1.57mmol) in t-butanol (15ml) and DMF (2ml) was added dropwise over 30 minutes to a solution of 9-[3-hydroxy-2-(phosphonomethoxy)propoxy]guanine (0.105g, 0.31mmol) and N,N-dicyclohexyl-4-morpholinocarboxamidine (0.092g, 0.31mmol) in 50% aqueous t-butanol (10ml). The reaction mixture was heated under reflux for 8 hours, allowed to cool to ambient temperature overnight and then filtered. The filtrate was evaporated to dryness, water added to the residue and the mixture filtered through a glass fibre filter paper. The filtrate was evaporated to dryness and the solid obtained purified on DEAE-Sephadex™ ($HCO_3^-$ form) eluting with a linear gradient of aqueous triethylammonium bicarbonate (pH 7.5) from 0.050M to 0.250M. The required component was found in the 0.15M fractions.

Relevant fractions were combined and evaporated to dryness co-evaporating with ethanol/water 3:1 (x3) when all traces of triethylamine were removed. The residue was converted into a sodium salt form by passing an aqueous solution through a column of DOWEX™ 50W-X8 ($Na^+$) resin and eluting with water. Relevant fractions were combined and lyophilized to leave the title compound as a white amorphous solid (58mg). UV: $\nu_{max}$ ($H_2O$) 253 ($\epsilon$ 11,600)nm. IR: $\nu_{max}$ (KBr) 3408, 3140, 2956, 1696, 1611, 1529, 1479, 1382,

1323, 1239, 1210, 1176, 1082, 1044, 1012, 961, 820, 792, 777 $cm^{-1}$. $^1H$ NMR: $\delta_H$ [$(CD_3)_2SO$] 3.40 (1H, dd, J=13.1 Hz, J=3.4Hz, $OC\underline{H}_BP$), 3.54 (1H, dd, J=13.1Hz, J=8.4Hz, $OC\underline{H}_AP$), 3.77 (1H, m, CH), 3.91 (1H, ddd, J=11.5Hz, J=15.0Hz, J=2.3Hz, $CHC\underline{H}_BOP$), 4.11 (1H, ddd, J=11.5Hz, J=9.7Hz, J=4.3Hz, $CHC\underline{H}_AOP$), 4.20 (1H, dd, J=10.6Hz, J=3.4Hz, $NOCH_B$), 4.29 (1H, dd, J=10.6Hz, J=6.6Hz, $NOCH_A$), 6.85 (2H, br.s, $D_2O$ exchangeable $NH_2$), 7.90 (1H, s, H-8), 10.99 1H, br.s, $D_2O$ exchangeable NH).

Example 16

2,6-Diamino-9-[3-hydroxy-2-(phosphonomethoxy)propoxy]purine

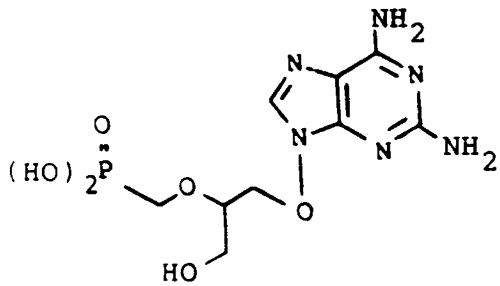

A solution of 9-[3-acetoxy-2-(diethoxyphosphorylmethoxy)propoxy]-6-chloro-2-formamidopurine (0.16g, 0.33mmol) in ethanolic ammonia (5ml) was heated in a sealed vessel at 120°C for 5 hours. The solution was allowed to cool to ambient temperature and the solvent was removed under reduced pressure to leave a brown residue. The residue was purified by preparative t.l.c. (dichloromethane:methanol 80:20) to give 2,6-diamino-9-[2-(diethoxyphosphorylmethoxy)-3-hydroxypropoxy]purine as a colourless gum (0.065g). The gum (0.06g, 0.15mmol) was dissolved in dry DMF (1ml) and trimethylsilyl bromide (0.3ml, 1.53mmol) added. After 3 hours at ambient temperature, the solvent was removed under reduced pressure and the residue obtained dissolved in 50% aqueous acetone. Evaporation of the solvent gave a solid which after crystallisation from hot water, gave the title compound as a cream solid (0.014g, 27%). $^1H$ NMR: $\delta_H$ [$(CD_3)_2SO$] 3.46-3.60 (2H, m, $C\underline{H}_2OH$), 3.65-3.85 (3H, m, $C\underline{H}OC\underline{H}_2P$), 4.24 (1H, m, $NOC\underline{H}_B$), 4.42 (1H, m, $NOC\underline{H}_A$), 6.03 (2H, br. s, $D_2O$ exchangeable $NH_2$), 6.88 (2H, br. s, $D_2O$ exchangeable $NH_2$), 8.02 (1H, s, H-8), 3.3 (br.s, $D_2O$ exchangeable OH, $PO(OH)_2$, water in solvent).
m/z (FAB, thioglycerol matrix) 335 ($MH^+$, 6%)

Antiviral Activity

1. Plaque Reduction Test for Herpes Simplex Viruses 1 and 2.

Cells (Vero or MRC-5) were grown to confluence in 24 well multi-dishes (well diameter = 1.5cm). The drained cell monolayers were each infected with approximately 50 infectious particles of herpes simplex virus 1 (HSV-1; strain HFEM) or herpes simplex virus 2 (HSV-2; strain MS) in 100µl of phosphate-buffered saline. The virus was absorbed for 1 hour at room temperature. After adsorption, residual inoculum was removed from each well and replaced with 0.5ml of Eagle's MEM containing 5% newborn calf serum and 0.9% agarose (A37). Once the agarose had set, dilutions of the test compound, which had been prepared in Eagle's MEM (containing 5% newborn calf serum), were added, each well receiving 0.5ml of liquid overlay. The test compound was diluted to give the following series of concentrations: 200, 60, 20, 6....0.06µg/ml; final concentrations in the assay ranged, therefore, between 100µg/ml and 0.03µg/ml. The infected cultures were incubated at 37°C in a humidified atmosphere of 5% $CO_2$ until plaques were clearly visible (2 or 3 days for Vero cells, usually 1 day for MRC-5 cells).

2. Plaque Reduction Test for Varicella Zoster Virus

MRC-5 cells were grown to confluence in 24 well multi-dishes (well diameter = 1.5cm). The drained cell monolayers were each infected with approximately 50 infectious particles of varicella zoster virus (VZV; Ellen strain) in 100µl of phosphate-buffered saline. The virus was adsorbed for 1 hour at room temperature.

After adsorption, residual inoculum was removed from each well and replaced with 0.5ml of Eagles's MEM containing 5% heat-activated foetal calf serum and 0.9% agarose (A37). Once the agarose had set, dilutions of the test compound, which had been prepared in Eagle's MEM (containing 5% heat-inactivated foetal calf serum), were added, each well receiving 0.5ml of liquid overlay. The test compound was diluted to give the following series of concentrations: 200, 60, 20, 6....0.06μg/ml; final concentrations in the assay ranged, therefore, between 100μg/ml and 0.03μg/ml. The infected cultures were incubated at 37°C in a humidified atmosphere of 5% $CO_2$ until plaques were clearly visible (5 or 6 days).

Cultures from 1 and 2 were fixed in formol saline, the agarose overlays were carefully washed off, and then the cell monolayers were stained with carbol fuchsin. A stereo microscope was used to count plaques. The $IC_{50}$ (concentration of drug which inhibits the number of plaques formed by 50% relative to the number of plaques observed in virus control monolayers) of the test compound was calculated. In addition, the monolayers were examined for evidence of drug-induced cytotoxicity; the minimum concentration at which cytotoxicity occurs was recorded.

3. CPE Inhibition Test (Established Monolayer) for Lentiviruses

$3 \times 10^4$ sheep choroid plexus (SCP) cells were plated into individual wells of a 96 well microtitre plate in 100μl of Eagle's MEM with Hanks' salts containing 10% heat inactivated foetal calf serum (FCS). When monolayers had become established (after 1 or 2 days growth) they were washed with 200μl of maintenance medium (Eagle's MEM with Hanks' salts containing 0.5% FCS) and infected with 100μl of visna virus (strain K184) in maintenance medium (20 $TCID_{50}$/ml). Test samples were diluted with maintenance medium in further 96 well microtitre plates over the range 200-0.06μg/ml by 3-fold dilution steps. 100μl of the diluted samples was then transferred directly onto virus-infected monolayers (final concentration range therefore 100-0.03μg/ml) and incubated at 37°C in a humidified atmosphere containing, 5% $CO_2$ until virus-induced CPE was maximal in the untreated virus-infected controls (usually 12-14 days). The plates were fixed with formal saline and stained with crystal violet. Virus-induced CPE was then scored microscopically and the minimum concentration of sample giving complete protection of the cell monolayers (MIC) determined.

Results

| Example No. | IC50 (μg/ml) Herpes Simplex virus Type 1 HFEM strain in Vero cells | Herpes Simplex virus Type 2 MS strain in MRC-5 cells | Varicella Zoster virus Ellen strain in MRC-5 cells | MIC (μg/ml) Visna Virus K184 strain in SCP cells |
|---|---|---|---|---|
| 1 | >100 | >100 | >100 | 100 |
| 2 | >100 | 69 | 80 | 3 |
| 3 | >100 | >100 | 51 | 10 |
| 4 | 1.1 | 0.08 | 0.06 | <0.003 |
| 5 | 59 | - | <3 | - |
| 7 | >100 | >100 | 66 | 0.3 |
| 8 | >100 | >100 | 60 | 1 |
| 10 | 17 | 7.3 | 21 | <0.003 |
| 11 | >100 | - | >100 | 30 |
| 12 | 24 | - | <3 | 0.3 |
| 13 | 3.7 | - | 0.5 | <0.03 |
| 14 | <3 | - | <0.03 | <0.03 |
| 15 | 29 | - | <3 | - |

Toxicity

At concentrations up to 30μg/ml, none of the compounds were cytotoxic for the cell monolayers used in any of the tests.

## Claims

**Claims for the following Contracting States : AT, BE, CH, DE, FR, GB, IT, LI, LU, NL, SE**

1. A compound of formula (I), or a pharmaceutically acceptable salt thereof:

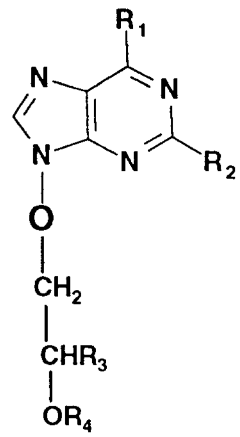

(I)

wherein
$R_1$ is hydroxy, amino, chloro or $OR_7$
wherein
$R_7$ is $C_{1-6}$ alkyl, phenyl or phenyl $C_{1-2}$ alkyl either of which phenyl moieties may be substituted by one or two substituents selected from halo, $C_{1-4}$ alkyl or $C_{1-4}$ alkoxy;
$R_2$ is amino or, when $R_1$ is hydroxy or amino, $R_2$ may also be hydrogen;
$R_3$ is hydrogen, hydroxymethyl or acyloxymethyl;
$R_4$ is a group of formula:

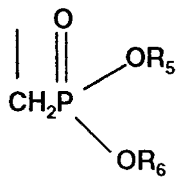

wherein
$R_5$ and $R_6$ are independently selected from hydrogen, $C_{1-6}$ alkyl and optionally substituted phenyl; or
$R_3$ and $R_4$ together are:

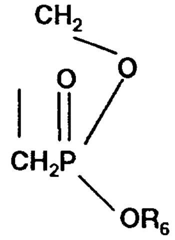

wherein $R_6$ is as defined above.

2. A compound according to claim 1 wherein $R_1$ is hydroxy and $R_2$ is amino.

**3.** A compound according to claim 1 wherein $R_1$ is amino and $R_2$ is hydrogen.

**4.** A compound according to any one of claims 1 to 3 wherein $R_3$ is hydroxymethyl.

**5.** A compound according to any one of claims 1 to 4 wherein $R_5$ and $R_6$ are both hydrogen.

**6.** A compound selected from the group consisting of:
9-[2-(diethoxyphosphorylmethoxy)ethoxy]adenine,
9-[2-(phosphonomethoxy)ethoxy]adenine,
9-[2-(diethoxyphosphorylmethoxy)ethoxy]guanine,
9-[2-(phosphonomethoxy)ethoxy]guanine,
9-[2-[ethoxy(hydroxy)phosphorylmethoxy)ethoxy]guanine,
9-[2-(diethoxyphosphorylmethoxy)-3-hydroxypropoxy]adenine,
9-[3-hydroxy-2-(phosphonomethoxy)propoxy]adenine,
9-[2-[ethoxy(hydroxy)phosphorylmethoxy]-3-hydroxypropoxy]adenine,
9-[2-(diethoxyphosphorylmethoxy)-3-hydroxypropoxy]guanine,
9-[3-hydroxy-2-(phosphonomethoxy)propoxy]guanine,
2,6-diamino-9-[2-(diethoxyphosphorylmethoxy)ethoxy]purine,
2,6-diamino-9-[2-phosphonomethoxy)ethoxy]purine,
2-amino-6-chloro-9-[2-(phosphonomethoxy)ethoxy]purine,
2-amino-6-methoxy-9-[2-(phosphonomethoxy)ethoxy]purine,
9-[(2-hydroxy-2-oxo-1,4,2-dioxaphosphorinan-5-yl)methoxy]guanine, and
2,6-diamino-9-[3-hydroxy-2-(phosphonomethoxy)propoxy]purine.

**7.** 9-[2-(Phosphonomethoxy)ethoxy]adenine.

**8.** A process for the preparation of a compound of formula (I), or a pharmaceutically acceptable salt thereof, as defined in claim 1, which process comprises either

i) imidazole ring closure of a compound of formula (II):

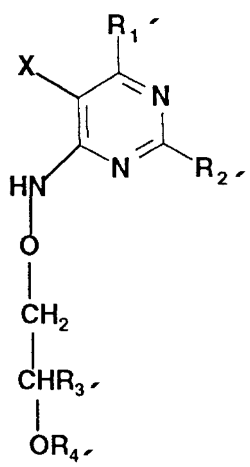

(II)

wherein X is formylamino; or

ii) pyrimidine ring closure of a compound of formula (III):

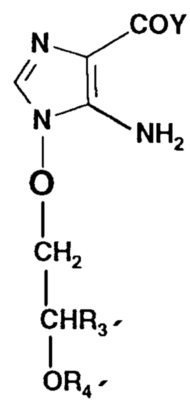

(III)

wherein Y is amino or $C_{1-6}$ alkoxy, with diethoxymethylacetate or triethylorthoformate, to give a compound of formula (I) wherein $R_1$ is hydroxy and $R_2$ is amino; or

iii) condensing a compound of formula (IV):

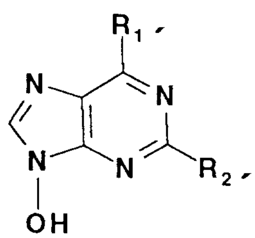

(IV)

with a side chain intermediate of formula (V):

$ZCH_2$-$CHR_3'OR_4'$ (V)

wherein Z is a leaving group;

and wherein, in formulae (II) to (V), $R_1'$, $R_2'$, $R_3'$, $R_4'$ are $R_1$, $R_2$, $R_3$ and $R_4$ respectively, as defined in claim 1, or groups or atoms convertible thereto; and thereafter, when desired or necessary, converting $R_1'$, $R_2'$, $R_3'$ and/or $R_4'$, when other than $R_1$, $R_2$, $R_3$ and/or $R_4$ to $R_1$, $R_2$, $R_3$ and/or $R_4$ respectively, and/or converting $R_1'$, $R_2'$, $R_3'$ and/or $R_4'$ when $R_1$, $R_2$, $R_3$ and/or $R_4$, to other $R_1$, $R_2$, $R_3$ and/or $R_4$.

**9.** An intermediate of formula (III) as defined in claim 8.

**10.** A compound selected from the group consisting of:
4-chloro-6-[2-(diethoxyphosphorylmethoxy)ethoxyamino]-5-formamidopyrimidine,
4-chloro-6-[2-(diethoxyphosphorylmethoxy)ethoxyamino]-2,5-diformamidopyrimidine,
6-[3-benzyloxy-2(diethoxyphosphorylmethoxy)propoxyamino]-4-chloro-5-formidopyrimidine,
6-[3-benzyloxy-2(diethoxyphosphorylmethoxy)propoxyamino]-4-chloro-2,5-diformamidopyrimidine,
6-[[3-acetoxy-2-(diethoxyphosphorylmethoxy)propoxy]amino]-4-chloro-5-formamidopyrimidine, and
6-[[3-acetoxy-2-(diethoxyphosphorylmethoxy)propoxy]amino]-4-chloro-2,5-diformamidopyrimidine.

**11.** An intermediate of formula (V) as defined in claim 8, wherein Z is replaced by an aminooxy group, and wherein $R_4'$ is $R_4$ as defined in claim 1.

**12.** A compound selected from:
diethyl(1-aminooxymethyl-2-benzyloxyethoxy)methylphosphonate, and
diethyl[2-acetoxy-1-(aminooxymethyl)ethoxy]methylphosphonate.

**13.** A pharmaceutical composition comprising a compound according to any one of claims 1 to 7, and a pharmaceutically acceptable carrier.

**14.** A compound according to any one of claims 1 to 7 for use as an active therapeutic substance.

**15.** A compound according to any one claims 1 to 7 for use in treating viral infections or in treating neoplastic diseases.

**16.** Use of a compound according to any one of claims 1 to 7 in the manufacture of a medicament for use in the treatment of viral infections or neoplastic diseases.

**17.** Use of 9-[2-phosphonomethoxy)ethoxy]adenine in the manufacture of a medicament for use in the treatment of human immunodeficiency virus infection.

**Claims for the following Contracting State : ES**

**1.** A process for the preparation of a compound of formula (I), or a pharmaceutically acceptable salt thereof

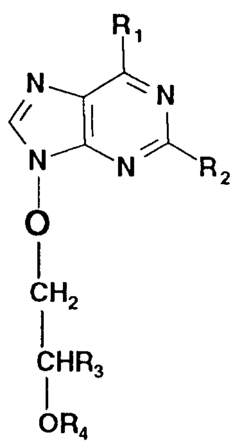

(I)

wherein

$R_1$ is hydroxy, amino, chloro or $OR_7$

wherein

$R_7$ is $C_{1-6}$ alkyl, phenyl or phenyl $C_{1-2}$ alkyl either of which phenyl moieties may be substituted by one or two substituents selected from halo, $C_{1-4}$ alkyl or $C_{1-4}$ alkoxy;

$R_2$ is amino or, when $R_1$ is hydroxy or amino, $R_2$ may also be hydrogen;

$R_3$ is hydrogen, hydroxymethyl or acyloxymethyl;

$R_4$ is a group of formula:

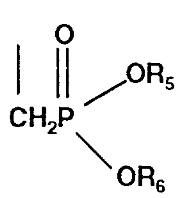

wherein

| | |
|---|---|
| $R_5$ and $R_6$ | are independently selected from hydrogen, $C_{1-6}$ alkyl and optionally substituted phenyl; or |
| $R_3$ and $R_4$ | together are: |

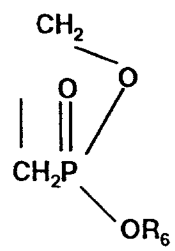

wherein $R_6$ is as defined above; which process comprises either

i) imidazole ring closure of a compound of formula (II):

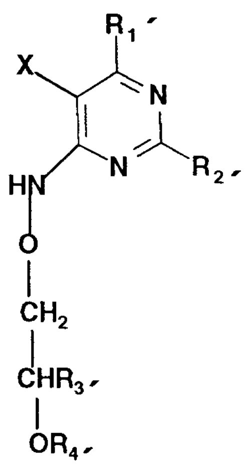

(II)

wherein X is formylamino; or

ii) pyrimidine ring closure of a compound of formula (III):

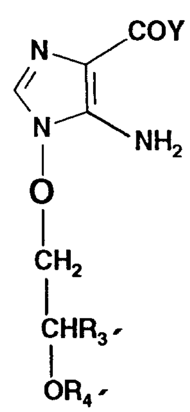

(III)

wherein Y is amino or $C_{1-6}$ alkoxy, with diethoxymethylacetate or triethylorthoformate to give a compound of formula (I) wherein $R_1$ is hydroxy and $R_2$ is amino; or

iii) condensing a compound of formula (IV):

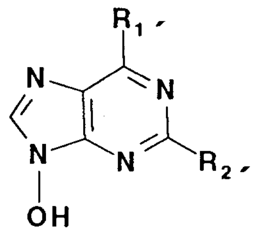

(IV)

with a side chain intermediate of formula (V):

$ZCH_2$-$CHR_3$'$OR_4$'    (V)

wherein Z is a leaving group;
and wherein, in formulae (II) to (V), $R_1$', $R_2$', $R_3$', $R_4$' are $R_1$, $R_2$, $R_3$ and $R_4$ respectively, or groups or atoms convertible thereto; and thereafter, when desired or necessary, converting $R_1$', $R_2$', $R_3$' and/or $R_4$', when other than $R_1$, $R_2$, $R_3$ and/or $R_4$ to $R_1$, $R_2$, $R_3$ and/or $R_4$ respectively, and/or converting $R_1$', $R_2$', $R_3$' and/or $R_4$' when $R_1$, $R_2$, $R_3$ and/or $R_4$, to other $R_1$, $R_2$, $R_3$ and/or $R_4$.

**2.** A process according to claim 1 wherein $R_1$ is hydroxy and $R_2$ is amino.

**3.** A process according to claim 1 wherein $R_1$ is amino and $R_2$ is hydrogen.

**4.** A process according to any one of claims 1 to 3 wherein $R_3$ is hydroxymethyl.

**5.** A process according to any one of claims 1 to 4 wherein $R_5$ and $R_6$ are both hydrogen.

**6.** A process according to claim 1 for the preparation of a compound selected from the group consisting of:
9-[2-(diethoxyphosphorylmethoxy)ethoxy]adenine,
9-[2-(phosphonomethoxy)ethoxy]adenine,
9-[2-(diethoxyphosphorylmethoxy)ethoxy]guanine,
9-[2-(phosphonomethoxy)ethoxy]guanine,
9-[2-[ethoxy(hydroxy)phosphorylmethoxy)ethoxy]guanine,
9-[2-(diethoxyphosphorylmethoxy)-3-hydroxypropoxy]adenine,
9-[3-hydroxy-2-(phosphonomethoxy)propoxy]adenine,
9-[2-[ethoxy(hydroxy)phosphorylmethoxy]-3-hydroxypropoxy]adenine,
9-[2-(diethoxyphosphorylmethoxy)-3-hydroxypropoxy]guanine,
9-[3-hydroxy-2-(phosphonomethoxy)propoxy]guanine,
2,6-diamino-9-[2-(diethoxyphosphorylmethoxy)ethoxy]purine,
2,6-diamino-9-[2-phosphonomethoxy)ethoxy]purine,
2-amino-6-chloro-9-[2-(phosphonomethoxy)ethoxy]purine,
2-amino-6-methoxy-9-[2-(phosphonomethoxy)ethoxy]purine,
9-[(2-hydroxy-2-oxo-1,4,2-dioxaphosphorinan-5-yl)methoxy]guanine, and
2,6-diamino-9-[3-hydroxy-2-(phosphonomethoxy)propoxy]purine.

**7.** A process according to claim 1 for the preparation of 9-[2-(phosphonomethoxy)ethoxy]adenine.

**8.** Use of a compound of formula (I) as defined in any one of claims 1 to 7 in the manufacture of a medicament for use in the treatment of viral infections or neoplastic diseases.

**9.** A process for the preparation of a pharmaceutical composition comprising the admixture of a compound of formula (I) as defined in any one of claims 1 to 7 and a pharmaceutical carrier, containing 50mg to 1g of active ingredient.

**10.** Use of 9-[2-phosphonomethoxy)ethoxy]adenine in the manufacture of a medicament for use in the treatment of human immunodeficiency virus infection.

**Claims for the following Contracting State : GR**

**1.** A compound of formula (I), or a pharmaceutically acceptable salt thereof:

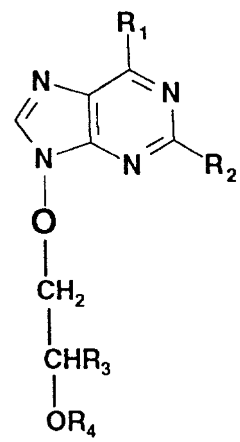

(I)

wherein

$R_1$ is hydroxy, amino, chloro or $OR_7$

wherein

$R_7$ is $C_{1-6}$ alkyl, phenyl or phenyl $C_{1-2}$ alkyl either of which phenyl moieties may be substituted by one or two substituents selected from halo, $C_{1-4}$ alkyl or $C_{1-4}$ alkoxy;

$R_2$ is amino or, when $R_1$ is hydroxy or amino, $R_2$ may also be hydrogen;

$R_3$ is hydrogen, hydroxymethyl or acyloxymethyl;

$R_4$ is a group of formula:

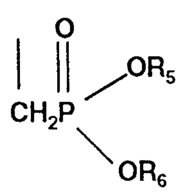

wherein

$R_5$ and $R_6$ are independently selected from hydrogen, $C_{1-6}$ alkyl and optionally substituted phenyl; or

$R_3$ and $R_4$ together are:

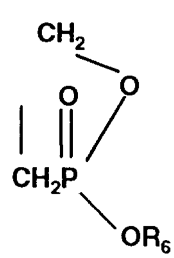

wherein $R_6$ is as defined above.

2. A compound according to claim 1 wherein $R_1$ is hydroxy and $R_2$ is amino.

3. A compound according to claim 1 wherein $R_1$ is amino and $R_2$ is hydrogen.

4. A compound according to any one of claims 1 to 3 wherein $R_3$ is hydroxymethyl.

5. A compound according to any one of claims 1 to 4 wherein $R_5$ and $R_6$ are both hydrogen.

6. A compound selected from the group consisting of:
9-[2-(diethoxyphosphorylmethoxy)ethoxy]adenine,
9-[2-(phosphonomethoxy)ethoxy]adenine,
9-[2-(diethoxyphosphorylmethoxy)ethoxy]guanine,
9-[2-(phosphonomethoxy)ethoxy]guanine,
9-[2-[ethoxy(hydroxy)phosphorylmethoxy)ethoxy]guanine,
9-[2-(diethoxyphosphorylmethoxy)-3-hydroxypropoxy]adenine,
9-[3-hydroxy-2-(phosphonomethoxy)propoxy]adenine,
9-[2-[ethoxy(hydroxy)phosphorylmethoxy]-3-hydroxypropoxy]adenine,
9-[2-(diethoxyphosphorylmethoxy)-3-hydroxypropoxy]guanine,
9-[3-hydroxy-2-(phosphonomethoxy)propoxy]guanine,
2,6-diamino-9-[2-(diethoxyphosphorylmethoxy)ethoxy]purine,
2,6-diamino-9-[2-phosphonomethoxy)ethoxy]purine,
2-amino-6-chloro-9-[2-(phosphonomethoxy)ethoxy]purine,
2-amino-6-methoxy-9-[2-(phosphonomethoxy)ethoxy]purine,
9-[(2-hydroxy-2-oxo-1,4,2-dioxaphosphorinan-5-yl)methoxy]guanine, and
2,6-diamino-9-[3-hydroxy-2-(phosphonomethoxy)propoxy]purine.

7. 9-[2-(Phosphonomethoxy)ethoxy]adenine.

8. A process for the preparation of a compound of formula (I), or a pharmaceutically acceptable salt thereof, as defined in claim 1, which process comprises either
i) imidazole ring closure of a compound of formula (II):

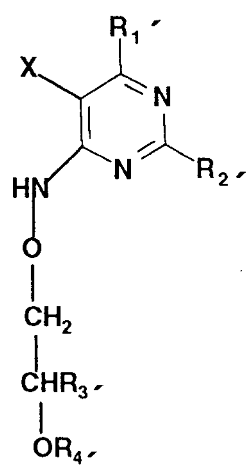

(II)

wherein X is formylamino; or

ii) pyrimidine ring closure of a compound of formula (III):

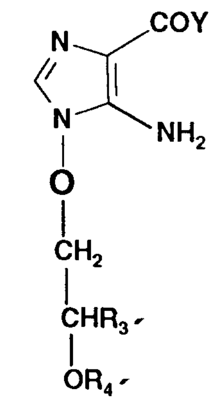

(III)

wherein Y is amino or $C_{1-6}$ alkoxy, with diethoxymethylacetate or triethylorthoformate, to give a compound of formula (I) wherein $R_1$ is hydroxy and $R_2$ is amino; or

iii) condensing a compound of formula (IV):

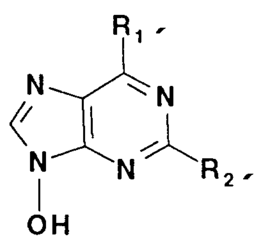

(IV)

with a side chain intermediate of formula (V):

$ZCH_2\text{-}CHR_3'OR_4'$ (V)

wherein Z is a leaving group;

and wherein, in formulae (II) to (V), $R_1'$, $R_2'$, $R_3'$, $R_4'$ are $R_1$, $R_2$, $R_3$ and $R_4$ respectively, as defined in claim 1, or groups or atoms convertible thereto; and thereafter, when desired or necessary, converting $R_1'$, $R_2'$, $R_3'$ and/or $R_4'$, when other than $R_1$, $R_2$, $R_3$ and/or $R_4$ to $R_1$, $R_2$, $R_3$ and/or $R_4$ respectively, and/or converting $R_1'$, $R_2'$, $R_3'$ and/or $R_4'$ when $R_1$, $R_2$, $R_3$ and/or $R_4$, to other $R_1$, $R_2$, $R_3$ and/or $R_4$.

**9.** An intermediate of formula (III) as defined in claim 8.

**10.** A compound selected from the group consisting of:
4-chloro-6-[2-(diethoxyphosphorylmethoxy)ethoxyamino]-5-formamidopyrimidine,
4-chloro-6-[2-(diethoxyphosphorylmethoxy)ethoxyamino]-2,5-diformamidopyrimidine,
6-[3-benzyloxy-2-(diethoxyphosphorylmethoxy)propoxyamino]-4-chloro-5-formidopyrimidine,
6-[3-benzyloxy-2-(diethoxyphosphorylmethoxy)propoxyamino]-4-chloro-2,5-diformamidopyrimidine,
6-[[3-acetoxy-2-(diethoxyphosphorylmethoxy)propoxy]amino]-4-chloro-5-formamidopyrimidine, and
6-[[3-acetoxy-2-(diethoxyphosphorylmethoxy)propoxy]amino]-4-chloro-2,5-diformamidopyrimidine.

**11.** An intermediate of formula (V) as defined in claim 8, wherein Z is replaced by an aminooxy group, and wherein $R_4'$ is $R_4$ as defined in claim 1.

**12.** A compound selected from:
diethyl(1-aminooxymethyl-2-benzyloxyethoxy)methylphosphonate, and
diethyl[2-acetoxy-1-(aminooxymethyl)ethoxy]methylphosphonate.

**13.** Use of a compound according to any one of claims 1 to 7 in the manufacture of a medicament for use in the treatment of viral infections or neoplastic diseases.

**14.** Use of 9-[2-phosphonomethoxy)ethoxy]adenine in the manufacture of a medicament for use in the treatment of human immunodeficiency virus infection.

**Patentansprüche**
**Patentansprüche für folgende Vertragsstaaten : AT, BE, CH, DE, FR, GB, IT, LI, LU, NL, SE**

**1.** Verbindung der Formel (I) oder ein pharmazeutisch verträgliches Salz davon:

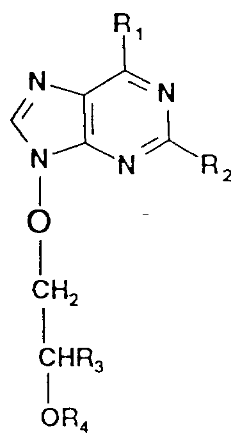

(I)

in der
$R_1$ eine Hydroxy-, Amino- oder Chlorgruppe oder einen Rest $OR_7$ darstellt, wobei $R_7$ einen $C_{1-6}$-Alkyl-, Phenyl- oder Phenyl-$C_{1-2}$-alkylrest darstellt, in dem jede der Phenyleinheiten durch einen oder zwei aus Halogen-, $C_{1-4}$-Alkyl- oder $C_{1-4}$-Alkoxyresten ausgewählten Substituenten substituiert sein kann;
$R_2$ eine Aminogruppe darstellt oder, falls $R_1$ eine Hydroxygruppe oder eine Aminogruppe darstellt, $R_2$ auch ein Wasserstoffatom darstellen kann;
$R_3$ ein Wasserstoffatom, eine Hydroxymethylgruppe oder einen Acyloxymethylrest darstellt;
$R_4$ einen Rest der Formel

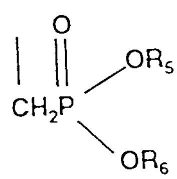

darstellt, wobei $R_5$ und $R_6$ unabhängig voneinander aus Wasserstoffatomen, $C_{1-6}$-Alkylresten und gegebenenfalls substituierten Phenylresten ausgewählt sind; oder
$R_3$ und $R_4$ zusammen den Rest

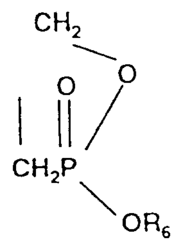

bilden, wobei $R_6$ wie vorstehend definiert ist.

2. Verbindung nach Anspruch 1, wobei $R_1$ eine Hydroxy- und $R_2$ eine Aminogruppe darstellt.

3. Verbindung nach Anspruch 1, wobei $R_1$ eine Aminogruppe und $R_2$ ein Wasserstoffatom darstellt.

4. Verbindung nach einem der Ansprüche 1 bis 3, wobei $R_3$ eine Hydroxymethylgruppe darstellt.

5. Verbindung nach einem der Ansprüche 1 bis 4, wobei sowohl $R_5$ als auch $R_6$ Wasserstoffatome darstellen.

6. Verbindung, ausgewählt aus der Gruppe:
9-[2-(Diethoxyphosphorylmethoxy)ethoxy]adenin,
9-[2-(Phosphonomethoxy)ethoxy]adenin,
9-[2-(Diethoxyphosphorylmethoxy)ethoxy]guanin,
9-[2-(Phosphonomethoxy)ethoxy]guanin,
9-(2-[Ethoxy(hydroxy)phosphorylmethoxy]ethoxy]guanin,
9-[2-(Diethoxyphosphorylmethoxy)-3-hydroxypropoxy]adenin,
9-[3-Hydroxy-2-(phosphonomethoxy)propoxy]adenin,
9-[2-Ethoxy(hydroxy)phosphorylmethoxy)-3-hydroxypropoxy]adenin,
9-[2-(Diethoxyphosphorylmethoxy)-3-hydroxypropoxy]guanin,
9-[3-Hydroxy-2-(phosphonomethoxy)propoxy]guanin,
2,6-Diamino-9-[2-(diethoxyphosphorylmethoxy)ethoxy]purin,
2,6-Diamino-9-[2-phosphonomethoxy)ethoxy]purin,
2-Amino-6-chlor-9-[2-phosphonomethoxy)ethoxy]purin,
2-Amino-6-methoxy-9-[2-phosphonomethoxy)ethoxy]purin,
9-[(2-Hydroxy-2-oxo-1,4,2-dioxaphosphorinan-5-yl)-methoxy]guanin und
2,6-Diamino-9-[3-hydroxy-2-(phosphonomethoxy)propoxy]purin.

7. 9-[2-(Phosphonomethoxy)ethoxy]adenin.

8. Verfahren zur Herstellung einer Verbindung der Formel (I) oder eines pharmazeutisch verträglichen Salzes davon, wie in Anspruch 1 definiert, das umfaßt entweder

i) einen Imidazol-Ringschluß der Verbindung der Formel (II):

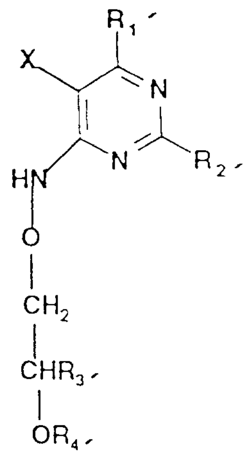

(II)

wobei X eine Formylaminogruppe darstellt; oder
ii) einen Pyrimidin-Ringschluß einer Verbindung der Formel (III):

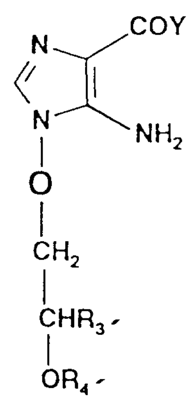

(III)

in der Y eine Aminogruppe oder einen $C_{1-6}$-Alkoxyrest darstellt, mit Diethoxymethylacetat oder Triethylorthoformiat, wobei eine Verbindung der Formel (I) erhalten wird, in der $R_1$ eine Hydroxy- und $R_2$ eine Aminogruppe darstellt; oder
iii) eine Kondensation einer Verbindung der Formel (IV):

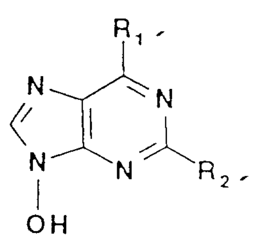

(IV)

mit einer Seitenketten-Zwischenstufe der Formel (V):

$ZCH_2\text{-}CHR_3'OR_4'$ (V)

in der Z eine Abgangsgruppe darstellt;
und wobei in den Formeln (II) bis (V) die Reste $R_1'$, $R_2'$, $R_3'$ bzw. $R_4'$ gleich den in Anspruch 1 definierten Resten $R_1$, $R_2$, $R_3$, bzw. $R_4$ sind oder Gruppen oder Atome darstellen, die in diese umzuwandeln sind; und
anschließend, falls erwünscht oder notwendig, Umwandlung der Reste $R_1'$, $R_2'$, $R_3'$ und/oder $R_4'$, falls es sich dabei um andere Reste als $R_1$, $R_2$, $R_3$ und/oder $R_4$ handelt, in die entsprechenden Reste $R_1$, $R_2$, $R_3$ und/oder $R_4$ und/oder Umwandlung der Reste $R_1'$, $R_2'$, $R_3'$ und/oder $R_4'$, falls es sich dabei um die Reste $R_1$, $R_2$, $R_3$ und/oder $R_4$ handelt, in andere Reste $R_1$, $R_2$, $R_3$ und/oder $R_4$.

**9.** Zwischenstufe der Formel (III), wie in Anspruch 8 definiert.

**10.** Verbindung, ausgewählt aus der Gruppe:
4-Chlor-6-[2-(diethoxyphosporylmethoxy)ethoxyamino]-5-formamidopyrimidin,
4-Chlor-6-[2-(diethoxyphosporylmethoxy)ethoxyamino]-2,5-diformamidopyrimidin,
6-[3-Benzyloxy-2-(diethoxyphosporylmethoxy)propoxyamino]-4-chlor-5-formamidopyrimidin,
6-[3-Benzyloxy-2-(diethoxyphosporylmethoxy)propoxyamino]-4-chlor-2,5-diformamidopyrimidin,
6-[[3-Acetoxy-2-(diethoxyphosporylmethoxy)propoxy]amino]-4-chlor-5-formamidopyrimidin und
6-[[3-Acetoxy-2-(diethoxyphosporylmethoxy)propoxy]amino]-4-chlor-2,5-diformamidopyrimidin.

**11.** Zwischenstufe der Formel (V), wie in Anspruch 8 definiert, wobei Z durch eine Aminooxygruppe ersetzt ist und $R_4'$ gleich $R_4$, wie in Anspruch 1 definiert, ist.

**12.** Verbindung, ausgewählt aus:
Diethyl(1-aminooxymethyl-2-benzyloxyethoxy)methylphosphonat und
Diethyl[2-acetoxy-1-(aminooxymethyl)ethoxy]methylphosphonat.

**13.** Arzneimittel, umfassend eine Verbindung nach einem der Ansprüche 1 bis 7 und einen pharmazeutisch verträglichen Träger.

**14.** Verbindung nach einem der Ansprüche 1 bis 7 zur Verwendung als therapeutischer Wirkstoff.

**15.** Verbindung nach einem der Ansprüche 1 bis 7 zur Verwendung bei der Behandlung von viralen Infektionen oder bei der Behandlung von neoplastischen Erkrankungen.

**16.** Verwendung einer Verbindung nach einem der Ansprüche 1 bis 7 bei der Herstellung eines Arzneimittels zur Verwendung bei der Behandlung von viralen Infektionen oder bei der Behandlung von neoplastischen Erkrankungen.

**17.** Verwendung von 9-[2-(Phosphonomethoxy)ethoxy]adenin bei der Herstellung eines Arzneimittels zur Verwendung bei der Behandlung von HIV(human immunodeficiency virus)-Infektionen.

**Patentansprüche für folgenden Vertragsstaat : ES**

**1.** Verfahren zur Herstellung einer Verbindung der Formel (I) oder eines pharmazeutisch verträglichen Salzes davon:

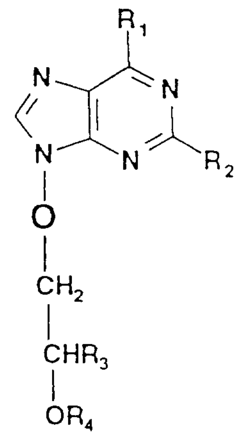

(I)

in der
$R_1$ eine Hydroxy-, Amino- oder Chlorgruppe oder einen Rest $OR_7$ darstellt, wobei $R_7$ einen $C_{1-6}$-Alkyl-, Phenyl- oder Phenyl-$C_{1-2}$-alkylrest darstellt, in dem jede der Phenyleinheiten durch einen oder zwei aus Halogen-, $C_{1-4}$-Alkyl- oder $C_{1-4}$-Alkoxyresten ausgewählten Substituenten substituiert sein kann;
$R_2$ eine Aminogruppe darstellt oder, falls $R_1$ eine Hydroxygruppe oder eine Aminogruppe darstellt, $R_2$ auch ein Wasserstoffatom darstellen kann;
$R_3$ ein Wasserstoffatom, eine Hydroxymethylgruppe oder einen Acyloxymethylrest darstellt;
$R_4$ einen Rest der Formel

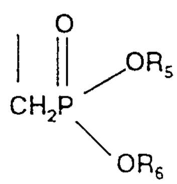

darstellt, wobei $R_5$ und $R_6$ unabhängig voneinander aus Wasserstoffatomen, $C_{1-6}$-Alkylresten und gegebenenfalls substituierten Phenylresten ausgewählt sind; oder
$R_3$ und $R_4$ zusammen den Rest

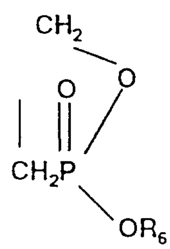

bilden, wobei $R_6$ wie vorstehend definiert ist; das umfaßt entweder

i) einen Imidazol-Ringschluß der Verbindung der Formel (II):

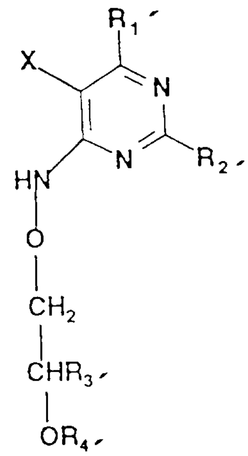

(II)

wobei X eine Formylaminogruppe darstellt; oder
ii) einen Pyrimidin-Ringschluß einer Verbindung der Formel (III):

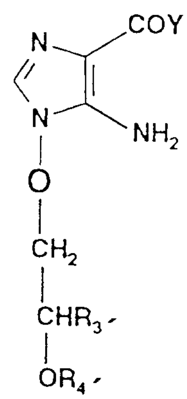

(III)

in der Y eine Aminogruppe oder einen $C_{1-6}$-Alkoxyrest darstellt, mit Diethoxymethylacetat oder Triethylorthoformiat, wobei eine Verbindung der Formel (I) erhalten wird, in der $R_1$ eine Hydroxy- und $R_2$ eine Aminogruppe darstellt; oder
iii) eine Kondensation einer Verbindung der Formel (IV):

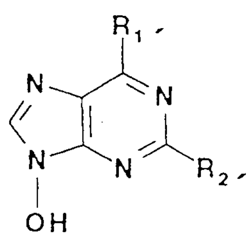

(IV)

mit einer Seitenketten-Zwischenstufe der Formel (V):

$ZCH_2$-$CHR_3$'$OR_4$'     (V)

in der Z eine Abgangsgruppe darstellt; und wobei in den Formeln (II) bis (V) die Reste $R_1$', $R_2$', $R_3$' bzw. $R_4$' gleich den in Anspruch 1 definierten Resten $R_1$, $R_2$, $R_3$, bzw. $R_4$ sind oder Gruppen oder Atome darstellen, die in diese umzuwandeln sind; und anschließend, falls erwünscht oder notwendig, Umwandlung der Reste $R_1$', $R_2$', $R_3$' und/oder $R_4$', falls es sich dabei um andere Reste als $R_1$, $R_2$, $R_3$ und/oder $R_4$ handelt, in die entsprechenden Reste $R_1$, $R_2$, $R_3$ und/oder $R_4$ und/oder Umwandlung der Reste $R_1$', $R_2$', $R_3$' und/oder $R_4$', falls es sich dabei um die Reste $R_1$, $R_2$, $R_3$ und/oder $R_4$ handelt, in andere Reste $R_1$, $R_2$, $R_3$ und/oder $R_4$.

**2.** Verfahren nach Anspruch 1, wobei $R_1$ eine Hydroxy- und $R_2$ eine Aminogruppe darstellt.

**3.** Verfahren nach Anspruch 1, wobei $R_1$ eine Aminogruppe und $R_2$ ein Wasserstoffatom darstellt.

**4.** Verfahren nach einem der Ansprüche 1 bis 3, wobei $R_3$ eine Hydroxymethylgruppe darstellt.

**5.** Verfahren nach einem der Ansprüche 1 bis 4, wobei sowohl $R_5$ als auch $R_6$ Wasserstoffatome darstellen.

**6.** Verfahren nach Anspruch 1 zur Herstellung einer Verbindung, ausgewählt aus der Gruppe:
9-[2-(Diethoxyphosphorylmethoxy)ethoxy]adenin,
9-[2-(Phosphonomethoxy)ethoxy]adenin,
9-[2-(Diethoxyphosphorylmethoxy)ethoxy]guanin,
9-[2-(Phosphonomethoxy)ethoxy]guanin,
9-[2-[Ethoxy(hydroxy)phosphorylmethoxy)ethoxy]guanin,
9-[2-(Diethoxyphosphorylmethoxy)-3-hydroxypropoxy]adenin,
9-[3-Hydroxy-2-(phosphonomethoxy)propoxy]adenin,
9-[2-Ethoxy(hydroxy)phosphorylmethoxy)-3-hydroxypropoxy]adenin,
9-[2-(Diethoxyphosphorylmethoxy)-3-hydroxypropoxy]guanin,
9-[3-Hydroxy-2-(phosphonomethoxy)propoxy]guanin,
2,6-Diamino-9-[2-(diethoxyphosphorylmethoxy)ethoxy]purin,
2,6-Diamino-9-[2-phosphonomethoxy)ethoxy]purin,
2-Amino-6-chlor-9-[2-phosphonomethoxy)ethoxy]purin,
2-Amino-6-methoxy-9-[2-phosphonomethoxy)ethoxy]purin,
9-[(2-Hydroxy-2-oxo-1,4,2-dioxaphosphorinan-5-yl)-methoxy]guanin und
2,6-Diamino-9-[3-hydroxy-2-(phosphonomethoxy)propoxy]purin.

**7.** Verfahren nach Anspruch 1 zur Herstellung von 9-[2-(Phosphonomethoxy)ethoxy]adenin.

**8.** Verwendung einer Verbindung der Formel (I), wie in einem der Ansprüche 1 bis 7 definiert, bei der Herstellung eines Arzneimittels zur Verwendung bei der Behandlung von viralen Infektionen oder von neoplastischen Erkrankungen.

**9.** Verfahren zur Herstellung eines Arzneimittels, umfassend das Zusammenmischen einer Verbindung der Formel (I), wie in einem der Ansprüche 1 bis 7 definiert, und eines pharmazeutischen Trägers, wobei das Mittel 50 mg bis 1 g des Wirkstoffs enthält.

**10.** Verwendung von 9-[2-(Phosphonomethoxy)ethoxy]adenin bei der Herstellung eines Arzneimittels zur Verwendung bei der Behandlung von HIV(human immunodeficiency virus)-Infektionen.

**Patentansprüche für folgenden Vertragsstaat : GR**

1. Verbindung der Formel (I) oder ein pharmazeutisch verträgliches Salz davon:

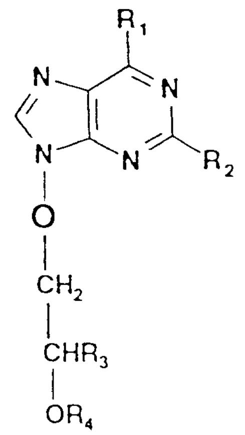

(I)

in der
$R_1$ eine Hydroxy-, Amino- oder Chlorgruppe oder einen Rest $OR_7$ darstellt, wobei $R_7$ einen $C_{1-6}$-Alkyl-, Phenyl- oder Phenyl-$C_{1-2}$-alkylrest darstellt, in dem jede der Phenyleinheiten durch einen oder zwei aus Halogen-, $C_{1-4}$-Alkyl- oder $C_{1-4}$-Alkoxyresten ausgewählten Substituenten substituiert sein kann;
$R_2$ eine Aminogruppe darstellt oder, falls $R_1$ eine Hydroxygruppe oder eine Aminogruppe darstellt, $R_2$ auch ein Wasserstoffatom darstellen kann;
$R_3$ ein Wasserstoffatom, eine Hydroxymethylgruppe oder einen Acyloxymethylrest darstellt;
$R_4$ einen Rest der Formel

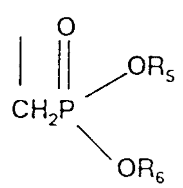

darstellt, wobei $R_5$ und $R_6$ unabhängig voneinander aus Wasserstoffatomen, $C_{1-6}$-Alkylresten und gegebenenfalls substituierten Phenylresten ausgewählt sind; oder
$R_3$ und $R_4$ zusammen den Rest

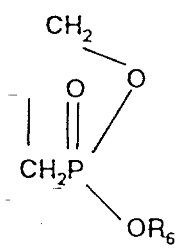

bilden, wobei $R_6$ wie vorstehend definiert ist.

2. Verbindung nach Anspruch 1, wobei $R_1$ eine Hydroxy- und $R_2$ eine Aminogruppe darstellt.

3. Verbindung nach Anspruch 1, wobei $R_1$ eine Aminogruppe und $R_2$ ein Wasserstoffatom darstellt.

**4.** Verbindung nach einem der Ansprüche 1 bis 3, wobei $R_3$ eine Hydroxymethylgruppe darstellt.

**5.** Verbindung nach einem der Ansprüche 1 bis 4, wobei sowohl $R_5$ als auch $R_6$ Wasserstoffatome darstellen.

**6.** Verbindung, ausgewählt aus der Gruppe:
9-[2-(Diethoxyphosphorylmethoxy)ethoxy]adenin,
9-[2-(Phosphonomethoxy)ethoxy]adenin,
9-[2-(Diethoxyphosphorylmethoxy)ethoxy]guanin,
9-[2-(Phosphonomethoxy)ethoxy]guanin,
9-[2-[Ethoxy(hydroxy)phosphorylmethoxy)ethoxy]guanin,
9-[2-(Diethoxyphosphorylmethoxy)-3-hydroxypropoxy]adenin,
9-[3-Hydroxy-2-(phosphonomethoxy)propoxy]adenin,
9-[2-Ethoxy(hydroxy)phosphorylmethoxy)-3-hydroxypropoxy]adenin,
9-[2-(Diethoxyphosphorylmethoxy)-3-hydroxypropoxy]guanin,
9-[3-Hydroxy-2-(phosphonomethoxy)propoxy]guanin,
2,6-Diamino-9-[2-(diethoxyphosphorylmethoxy)ethoxy]purin,
2,6-Diamino-9-[2-phosphonomethoxy)ethoxy]purin,
2-Amino-6-chlor-9-[2-phosphonomethoxy)ethoxy]purin,
2-Amino-6-methoxy-9-[2-phosphonomethoxy)ethoxy]purin,
9-[(2-Hydroxy-2-oxo-1,4,2-dioxaphosphorinan-5-yl)-methoxy]guanin und
2,6-Diamino-9-[3-hydroxy-2-(phosphonomethoxy)propoxy]purin.

**7.** 9-[2-(Phosphonomethoxy)ethoxy]adenin.

**8.** Verfahren zur Herstellung einer Verbindung der Formel (I) oder eines pharmazeutisch verträglichen Salzes davon, wie in Anspruch 1 definiert, das umfaßt entweder

i) einen Imidazol-Ringschluß der Verbindung der Formel (II):

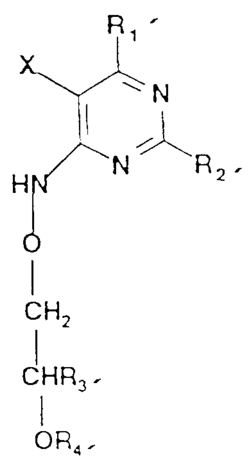

(II)

wobei X eine Formylaminogruppe darstellt; oder

ii) einen Pyrimidin-Ringschluß einer Verbindung der Formel (III):

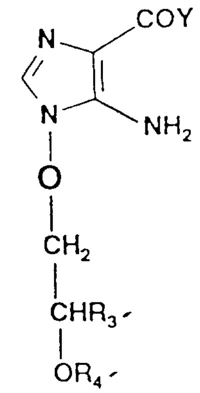

(III)

in der Y eine Aminogruppe oder einen $C_{1-6}$-Alkoxyrest darstellt, mit Diethoxymethylacetat oder Triethylorthoformiat, wobei eine Verbindung der Formel (I) erhalten wird, in der $R_1$ eine Hydroxy- und $R_2$ eine Aminogruppe darstellt; oder

iii) eine Kondensation einer Verbindung der Formel (IV):

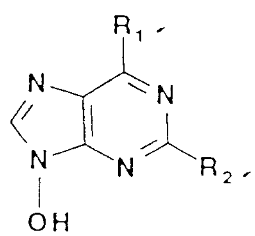

(IV)

mit einer Seitenketten-Zwischenstufe der Formel (V):

$ZCH_2$-$CHR_3'OR_4'$ (V)

in der Z eine Abgangsgruppe darstellt;

und wobei in den Formeln (II) bis (V) die Reste $R_1'$, $R_2'$, $R_3'$ bzw. $R_4'$ gleich den in Anspruch 1 definierten Resten $R_1$, $R_2$, $R_3$, bzw. $R_4$ sind oder Gruppen oder Atome darstellen, die in diese umzuwandeln sind; und

anschließend, falls erwünscht oder notwendig, Umwandlung der Reste $R_1'$, $R_2'$, $R_3'$ und/oder $R_4'$, falls es sich dabei um andere Reste als $R_1$, $R_2$, $R_3$ und/oder $R_4$ handelt, in die entsprechenden Reste $R_1$, $R_2$, $R_3$ und/oder $R_4$ und/oder Umwandlung der Reste $R_1'$, $R_2'$, $R_3'$ und/oder $R_4'$, falls es sich dabei um die Reste $R_1$, $R_2$, $R_3$ und/oder $R_4$ handelt, in andere Reste $R_1$, $R_2$, $R_3$ und/oder $R_4$.

**9.** Zwischenstufe der Formel (III), wie in Anspruch 8 definiert.

**10.** Verbindung, ausgewählt aus der Gruppe:
4-Chlor-6-[2-(diethoxyphosporylmethoxy)ethoxyamino]-5-formamidopyrimidin,
4-Chlor-6-[2-(diethoxyphosporylmethoxy)ethoxyamino]-2,5-diformamidopyrimidin,
6-[3-Benzyloxy-2-(diethoxyphosporylmethoxy)propoxyamino]-4-chlor-5-formamidopyrimidin,
6-[3-Benzyloxy-2-(diethoxyphosporylmethoxy)propoxyamino]-4-chlor-2,5-diformamidopyrimidin,
6-[[3-Acetoxy-2-(diethoxyphosporylmethoxy)propoxy]amino]-4-chlor-5-formamidopyrimidin und

6-[[3-Acetoxy-2-(diethoxyphosporylmethoxy)propoxy]amino]-4-chlor-2,5-diformamidopyrimidin.

**11.** Zwischenstufe der Formel (V), wie in Anspruch 8 definiert, wobei Z durch eine Aminooxygruppe ersetzt ist und $R_4$' gleich $R_4$, wie in Anspruch 1 definiert, ist.

**12.** Verbindung, ausgewählt aus:
Diethyl(1-aminooxymethyl-2-benzyloxyethoxy)methylphosphonat und
Diethyl[2-acetoxy-1-(aminooxymethyl)ethoxy]methylphosphonat.

**13.** Verwendung einer Verbindung nach einem der Ansprüche 1 bis 7 bei der Herstellung eines Arzneimittels zur Verwendung bei der Behandlung von viralen Infektionen oder von neoplastischen Erkrankungen.

**14.** Verwendung von 9-[2-(Phosphonomethoxy)ethoxy]adenin bei der Herstellung eines Arzneimittels zur Verwendung bei der Behandlung von HIV(human immunodeficiency virus)-Infektionen.

**Revendications**
**Revendications pour les Etats contractant suivants : AT, BE, CH, DE, FR, GB, IT, LI, LU, NL, SE**

**1.** Composé de formule (I)ou sel de celui-ci acceptable du point de vue pharmaceutique:

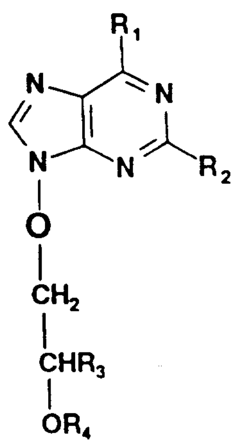

(I)

dans laquelle :

$R_1$ est un groupe hydroxy, amino, un atome de chlore ou un groupe $OR_7$ dans lequel:

$R_7$ est un groupe alkyle en $C_{1-6}$, phényle ou phénylalkyle en $C_{1-2}$, l'une ou l'autre partie phényle pouvant porter un ou deux substituants choisis parmi un atome d'halogène, un groupe alkyle en $C_{1-4}$ ou alcoxy en $C_{1-4}$;

$R_2$ est un groupe amino ou, lorsque $R_1$ est un groupe hydroxy ou amino, $R_2$ peut aussi être un atome d'hydrogène;

$R_3$ est un atome d'hydrogène, un groupe hydroxyméthyle ou acyloxyméthyle;

$R_4$ est un groupe de formule :

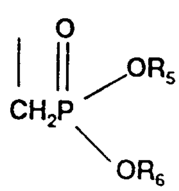

dans laquelle :

$R_5$ et $R_6$ sont choisis indépendamment parmi un atome d'hydrogène, un groupe alkyle en $C_{1-6}$ et un groupe phényle éventuellement substitué; ou

$R_3$ et $R_4$ forment ensemble un groupe de formule :

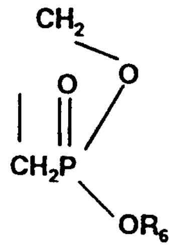

dans laquelle $R_6$ est tel que défini plus haut.

2. Composé suivant la revendication 1, dans lequel $R_1$ est un groupe hydroxy et $R_2$ est un groupe amino.

3. Composé suivant la revendication 1, dans lequel $R_1$ est un groupe amino et $R_2$ est un atome d'hydrogène.

4. Composé suivant l'une quelconque des revendications 1 à 3, dans lequel $R_3$ est un groupe hydroxyméthyle.

5. Composé suivant l'une quelconque des revendications 1 à 4, dans lequel $R_5$ et $R_6$ sont chacun un atome d'hydrogène.

6. Composé sélectionné dans le groupe consistant en :
9-[2-(diéthoxyphosphorylméthoxy)-éthoxy]-adénine,
9-[2-(phosphonométhoxy)-éthoxy]-adénine,
9-[2-(diéthoxyphosphorylméthoxy)-éthoxy]-guanine,
9-[2-(phosphonométhoxy)-éthoxy]-guanine,
9-[2-(éthoxy-(hydroxy)-phosphorylméthoxy)-éthoxy]-guanine,
9-[2-(diéthoxyphosphorylméthoxy)-3-hydroxypropoxy]-adénine,
9-[3-hydroxy-2-(phosphonométhoxy)-propoxy]-adénine,
9-[2-[éthoxy(hydroxy)-phosphorylméthoxy]-3-hydroxypropoxy]-adénine,
9-[2-(diéthoxyphosphorylméthoxy)-3-hydroxypropoxy]-guanine,
9-[3-hydroxy-2-(phosphonométhoxy)-propoxy]-guanine,
2,6-diamino-9-[2-(diéthoxyphosphorylméthoxy)-éthoxy]-purine,
2,6-diamino-9-[2-(phosphonométhoxy)-éthoxy]-purine,
2-amino-6-chloro-9-[2-(phosphonométhoxy)-éthoxy]-purine,
2-amino-6-méthoxy-9-[2-(phosphonométhoxy)-éthoxy]-purine,
9-[(2-hydroxy-2-oxo-1,4,2-dioxaphosphorinan-5-yl)-méthoxy]-guanine, et
2,6-diamino-9-[3-hydroxy-2-(phosphonométhoxy)-propoxy]-purine.

7. Composé suivant la revendication 1, qui est la 9-[2-(phosphonométhoxy)-éthoxy]-adénine.

8. Procédé pour la préparation d'un composé de formule (I) ou d'un sel de celui-ci acceptable du point de vue pharmaceutique suivant la revendication 1, qui comprend, soit

(i) la fermeture d'un cycle imidazole d'un composé de formule (II) :

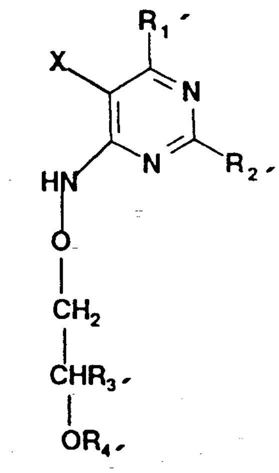

(II)

dans laquelle X est un groupe formylamino; soit
(ii) la fermeture d'un cycle pyrimidine d'un composé de formule (III) :

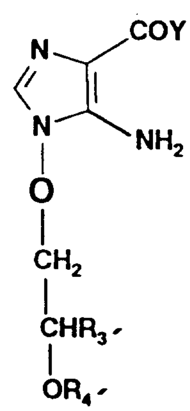

(III)

dans laquelle Y est un groupe amino ou alcoxy en $C_{1-6}$, avec de l'acétate de diéthoxyméthyle ou de l'orthoformiate de triéthyle, pour donner un composé de formule (I) dans laquelle $R_1$ est un groupe hydroxy et $R_2$ est un groupe amino; soit
(iii) la condensation d'un composé de formule (IV) :

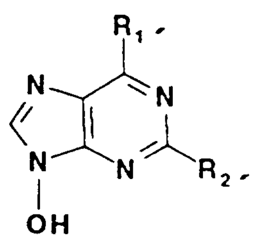

(IV)

avec un intermédiaire de chaîne latérale de formule (V) :

$ZCH_2\text{-}CHR_3'\text{-}OR_4'$ (V)

dans laquelle Z est un groupe mobile;
et où, dans les formules (II) à (V), $R_1'$, $R_2'$, $R_3'$, $R_4'$ sont $R_1$, $R_2$, $R_3$ et $R_4$ respectivement tels que définis dans la revendication 1, ou des groupes ou des atomes pouvant être convertis en ceux-ci; et

si cela est désiré ou nécessaire, la conversion de $R_1'$, $R_2'$, $R_3'$ et/ou $R_4'$ lorsqu'ils sont autres que $R_1$, $R_2$, $R_3$ et/ou $R_4$ en $R_1$, $R_2$, $R_3$ et/ou $R_4$ respectivement, et/ou la conversion de $R_1'$, $R_2'$, $R_3'$ et/ou $R_4'$ lorsqu'ils sont $R_1$, $R_2$, $R_3$ et/ou $R_4$ en d'autres $R_1$, $R_2$, $R_3$ et/ou $R_4$.

**9.** Intermédiaire de formule (III) tel que défini dans la revendication 8.

**10.** Composé choisi dans le groupe consistant en :
4-chloro-6-[2-(diéthoxyphosphorylméthoxy)-éthoxyamino]-5-formamidopyrimidine,
4-chloro-6-[2(diéthoxyphosphorylméthoxy)-éthoxyamino]2,5-diformamidopyrimidine,
4-chloro-6-[3-benzyloxy-2-(diéthoxyphosphorylméthoxy)-propoxyamino]-5-formamidopyrimidine,
4-chloro-6-[3-benzyloxy-2-(diéthoxyphosphorylméthoxy)-propoxyamino]-2,5-diformamidopyrimidine,
4-chloro-6-[[3-acétoxy-2-(diéthoxyphosphorylméthoxy)-propoxy]-amino]-5-formamidopyrimidine et
4-chloro-6-[[3-acétoxy-2-(diéthoxyphosphorylméthoxy)-propoxy]-amino]-2,5-diformamidopyrimidine.

**11.** Intermédiaire de formule (V) tel que défini dans la revendication 8 dans lequel Z est remplacé par un groupe aminooxy et dans lequel $R_4'$ est $R_4$ tel que défini dans la revendication 1.

**12.** Composé choisi parmi:
le (1-aminooxyméthyl-2-benzyloxyéthoxy)-méthylphosphonate de diéthyle et
le [2-acétoxy-1-(aminooxyméthyl)-éthoxy]-méthylphosphonate de diéthyle.

**13.** Composition pharmaceutique comprenant un composé suivant l'une quelconque des revendications 1 à 7 et un support acceptable du point de vue pharmaceutique.

**14.** Composé suivant l'une quelconque des revendications 1 à 7, utile comme substance thérapeutique active.

**15.** Composé suivant l'une quelconque des revendications 1 à 7, utile dans le traitement d'infections virales ou dans le traitement de maladies néoplastiques.

**16.** Utilisation d'un composé suivant l'une quelconque des revendications 1 à 7, dans la fabrication d'un médicament utile dans le traitement d'infections virales ou de maladies néoplastiques.

**17.** Utilisation de la 9-[2-(phosphonométhoxy)-éthoxy]-adénine dans la fabrication d'un médicament utile dans le traitement d'infection par le virus de l'immunodéficience humaine.

**Revendications pour l'Etat contractant suivant : ES**

**1.** Procédé pour la préparation d'un composé de formule (I) ou d'un sel de celui-ci acceptable du point de vue pharmaceutique:

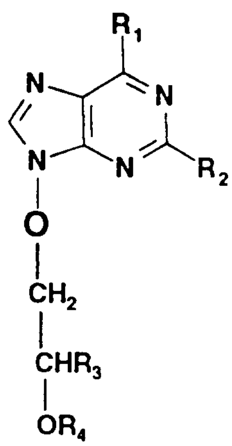

(I)

dans laquelle :

$R_1$ est un groupe hydroxy, amino, un atome de chlore ou un groupe $OR_7$ dans lequel:

$R_7$ est un groupe alkyle en $C_{1-6}$, phényle ou phénylalkyle en $C_{1-2}$, l'une ou l'autre partie phényle pouvant porter un ou deux substituants choisis parmi un atome d'halogène, un groupe alkyle en $C_{1-4}$ ou alcoxy en $C_{1-4}$;

$R_2$ est un groupe amino ou, lorsque $R_1$ est un groupe hydroxy ou amino, $R_2$ peut aussi être un atome d'hydrogène;

$R_3$ est un atome d'hydrogène, un groupe hydroxyméthyle ou acyloxyméthyle;

$R_4$ est un groupe de formule :

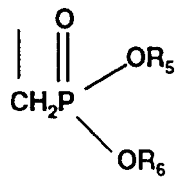

dans laquelle :

$R_5$ et $R_6$ sont choisis indépendamment parmi un atome d'hydrogène, un groupe alkyle en $C_{1-6}$ et un groupe phényle éventuellement substitué; ou

$R_3$ et $R_4$ forment ensemble un groupe de formule :

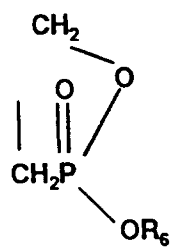

dans laquelle $R_6$ est tel que défini plus haut, qui comprend, soit

(i) la fermeture d'un cycle imidazole d'un composé de formule (II) :

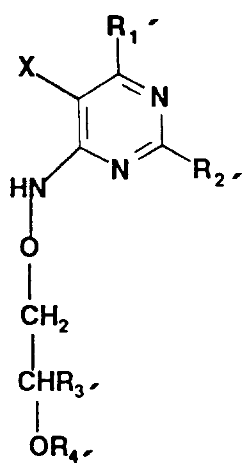

(II)

dans laquelle X est un groupe formylamino; soit

(ii) la fermeture d'un cycle pyrimidine d'un composé de formule (III) :

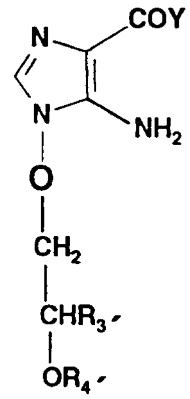

(III)

dans laquelle Y est un groupe amino ou alcoxy en $C_{1-6}$, avec de l'acétate de diéthoxyméthyle ou de l'orthoformiate de triéthyle, pour donner un composé de formule (I) dans laquelle $R_1$ est un groupe hydroxy et $R_2$ est un groupe amino; soit
(iii) la condensation d'un composé de formule (IV) :

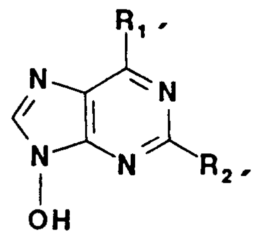

(IV)

avec un intermédiaire de chaîne latérale de formule (V) :

$ZCH_2$-$CHR_3$'-$OR_4$'   (V)

dans laquelle Z est un groupe mobile;
et où dans les formules (II) à (V), $R_1$', $R_2$', $R_3$', $R_4$' sont $R_1$, $R_2$, $R_3$ et $R_4$ respectivement tels que définis dans la revendication 1, ou des groupes ou des atomes pouvant être convertis en ceux-ci; et ensuite, si cela est désiré ou nécessaire, la conversion de $R_1$', $R_2$', $R_3$' et/ou $R_4$' lorsqu'ils sont autres que $R_1$, $R_2$, $R_3$ et/ou $R_4$ en $R_1$, $R_2$, $R_3$ et/ou $R_4$ respectivement, et/ou la conversion de $R_1$', $R_2$', $R_3$' et/ou $R_4$' lorsqu'ils sont $R_1$, $R_2$, $R_3$ et/ou $R_4$ en d'autres $R_1$, $R_2$, $R_3$ et/ou $R_4$.

**2.** Procédé suivant la revendication 1, dans lequel $R_1$ est un groupe hydroxy et $R_2$ est un groupe amino.

**3.** Procédé suivant la revendication 1, dans lequel $R_1$ est un groupe amino et $R_2$ est un atome d'hydrogène.

**4.** Procédé suivant l'une quelconque des revendications 1 à 3, dans lequel $R_3$ est un groupe hydroxyméthyle.

**5.** Procédé suivant l'une quelconque des revendications 1 à 4, dans lequel $R_5$ et $R_6$ sont chacun un atome d'hydrogène.

**6.** Procédé suivant la revendication 1, pour la préparation d'un composé sélectionné dans le groupe consistant en :
9-[2-(diéthoxyphosphorylméthoxy)-éthoxy]-adénine,
9-[2-(phosphonométhoxy)-éthoxy]-adénine,

9-[2-(diéthoxyphosphorylméthoxy)-éthoxy]-guanine,
9-[2-(phosphonométhoxy)-éthoxy]-guanine,
9-[2-(éthoxy-(hydroxy)-phosphorylméthoxy)-éthoxy]-guanine,
9-[2-(diéthoxyphosphorylméthoxy)-3-hydroxypropoxy]-adénine,
9-[3-hydroxy-2-(phosphonométhoxy)-propoxy]-adénine,
9-[2-[éthoxy(hydroxy)-phosphorylméthoxy]-3-hydroxypropoxy]-adénine,
9-[2-(diéthoxyphosphorylméthoxy)-3-hydroxypropoxy]-guanine,
9-[3-hydroxy-2-(phosphonométhoxy)-propoxy]-guanine,
2,6-diamino-9-[2-(diéthoxyphosphorylméthoxy)-éthoxy]-purine,
2,6-diamino-9-[2-(phosphonométhoxy)-éthoxy]-purine,
2-amino-6-chloro-9-[2-(phosphonométhoxy)-éthoxy]-purine,
2-amino-6-méthoxy-9-[2-(phosphonométhoxy)-éthoxy]-purine,
9-[(2-hydroxy-2-oxo-1,4,2-dioxaphosphorinan-5-yl)-méthoxy]-guanine, et
2,6-diamino-9-[3-hydroxy-2-(phosphonométhoxy)-propoxy]-purine,

**7.** Procédé suivant la revendication 1, pour la préparation de 9-[2-(phosphonométhoxy)-éthoxy]-adénine.

**8.** Utilisation d'un composé suivant l'une quelconque des revendications 1 à 7, dans la fabrication d'un médicament utile dans le traitement d'infections virales ou de maladies néoplastiques.

**9.** Procédé pour la préparation d'une composition pharmaceutique comprenant un mélange d'un composé de formule (I) suivant l'une quelconque des revendications 1 à 7 et d'un support pharmaceutique, contenant 50 mg à 1 g de composant actif.

**10.** Utilisation de la 9-[2-(phosphonométhoxy)-éthoxy]-adénine dans la fabrication d'un médicament utile dans le traitement d'infection par le virus de l'immunodéficience humaine.

**Revendications pour l'Etat contractant suivant : GR**

**1.** Composé de formule (I) ou sel de celui-ci acceptable du point de vue pharmaceutique:

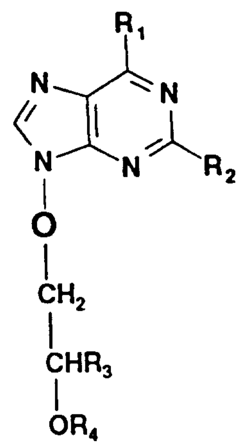

(I)

dans laquelle :

- $R_1$ est un groupe hydroxy, amino, un atome de chlore ou un groupe $OR_7$ dans lequel:
- $R_7$ est un groupe alkyle en $C_{1-6}$, phényle ou phénylalkyle en $C_{1-2}$,l'une ou l'autre partie phényle pouvant porter un ou deux substituants choisis parmi un atome d'halogène, un groupe alkyle en $C_{1-4}$ ou alcoxy en $C_{1-4}$;
- $R_2$ est un groupe amino ou, lorsque $R_1$ est un groupe hydroxy ou amino, $R_2$ peut aussi être un atome d'hydrogène;
- $R_3$ est un atome d'hydrogène, un groupe hydroxyméthyle ou acyloxyméthyle;
- $R_4$ est un groupe de formule :

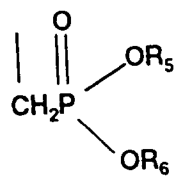

dans laquelle :

$R_5$ et $R_6$ sont choisis indépendamment parmi un atome d'hydrogène, un groupe alkyle en $C_{1-6}$ et un groupe phényle éventuellement substitué; ou

$R_3$ et $R_4$ forment ensemble un groupe de formule :

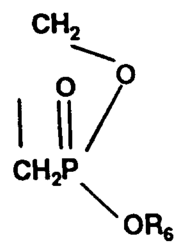

dans laquelle $R_6$ est tel que défini plus haut.

2. Composé suivant la revendication 1, dans lequel $R_1$ est un groupe hydroxy et $R_2$ est un groupe amino.

3. Composé suivant la revendication 1, dans lequel $R_1$ est un groupe amino et $R_2$ est un atome d'hydrogène.

4. Composé suivant l'une quelconque des revendications 1 à 3, dans lequel $R_3$ est un groupe hydroxyméthyle.

5. Composé suivant l'une quelconque des revendications 1 à 4, dans lequel $R_5$ et $R_6$ sont chacun un atome d'hydrogène.

6. Composé sélectionné dans le groupe consistant en :
9-[2-(diéthoxyphosphorylméthoxy)-éthoxy]-adénine,
9-[2-(phosphonométhoxy)-éthoxy]-adénine,
9-[2-(diéthoxyphosphorylméthoxy)-éthoxy]-guanine,
9-[2-(phosphonométhoxy)-éthoxy]-guanine,
9-[2-(éthoxy-(hydroxy)-phosphorylméthoxy)-éthoxy]-guanine,
9-[2-(diéthoxyphosphorylméthoxy)-3-hydroxypropoxy]-adénine,
9-[3-hydroxy-2-(phosphonométhoxy)-propoxy]-adénine,
9-[2-[éthoxy(hydroxy)-phosphorylméthoxy]-3-hydroxypropoxy]-adénine,
9-[2-(diéthoxyphosphorylméthoxy)-3-hydroxypropoxy]-guanine,
9-[3-hydroxy-2-(phosphonométhoxy)-propoxy]-guanine,
2,6-diamino-9-[2-(diéthoxyphosphorylméthoxy)-éthoxy]-purine,
2,6-diamino-9-[2-(phosphonométhoxy)-éthoxy]-purine,
2-amino-6-chloro-9-[2-(phosphonométhoxy)-éthoxy]-purine,
2-amino-6-méthoxy-9-[2-(phosphonométhoxy)-éthoxy]-purine,
9-[(2-hydroxy-2-oxo-1,4,2-dioxaphosphorinan-5-yl)-méthoxy]-guanine, et
2,6-diamino-9-[3-hydroxy-2-(phosphonométhoxy)-propoxy]-purine.

7. Composé suivant la revendication 1 qui est la 9-[2-(phosphonométhoxy)-éthoxy]-adénine.

8. Procédé pour la préparation d'un composé de formule (I) ou d'un sel de celui-ci acceptable du point de vue pharmaceutique suivant la revendication 1, qui comprend, soit

(i) la fermeture d'un cycle imidazole d'un composé de formule (II) :

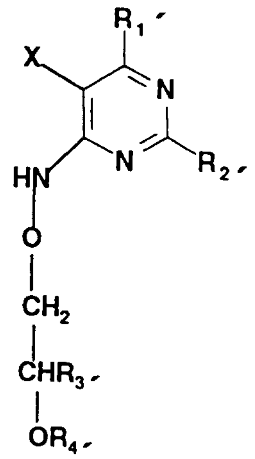

(II)

dans laquelle X est un groupe formylamino; soit

(ii) la fermeture d'un cycle pyrimidine d'un composé de formule (III) :

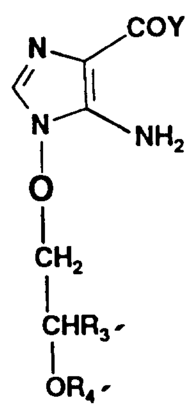

(III)

dans laquelle Y est un groupe amino ou alcoxy en $C_{1-6}$, avec de l'acétate de diéthoxyméthyle ou de l'orthoformiate de triéthyle, pour donner un composé de formule (I) dans laquelle $R_1$ est un groupe hydroxy et $R_2$ est un groupe amino; soit

(iii) la condensation d'un composé de formule (IV) :

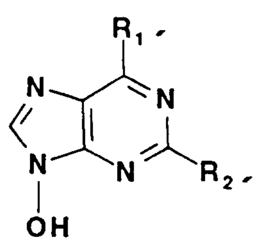

(IV)

avec un intermédiaire de chaîne latérale de formule (V) :

$ZCH_2\text{-}CHR_3'\text{-}OR_4'$ (V)

dans laquelle Z est un groupe mobile;

et dans lesquelles formules (II) à (V), $R_1'$, $R_2'$, $R_3'$, R4' sont $R_1$, $R_3$, $R_3$ et $R_4$ respectivement tels que définis dans la revendication 1, ou des groupes ou des atomes pouvant être convertis en ceux-ci; et ensuite, si cela est désiré ou nécessaire, la conversion de $R_1'$, $R_2'$, $R_3'$ et/ou $R_4'$ lorsqu'ils sont autres que $R_1$, $R_2$, $R_3$ et/ou $R_4$ en $R_1$, $R_2$, $R_3$ et/ou $R_4$ respectivement, et/ou la conversion de $R_1'$, $R_2'$, $R_3'$ et/ou $R_4'$ lorsqu'ils sont $R_1$, $R_2$, $R_3$ et/ou $R_4$ en d'autres $R_1$, $R_2$, $R_3$ et/ou $R_4$.

**9.** Intermédiaire de formule (III) tel que défini dans la revendication 8.

**10.** Composé choisi dans le groupe consistant en :
4-chloro-6-[2-(diéthoxyphosphorylméthoxy)-éthoxyamino]-5-formamidopyrimidine,
4-chloro-6-[2-(diéthoxyphosphorylméthoxy)-éthoxyamino]-2,5-diformamidopyrimidine,
4-chloro-6-[3-benzyloxy-2-(diéthoxyphosphorylméthoxy)-propoxyamino]-5-formamidopyrimidine,
4-chloro-6-[3-benzyloxy-2-(diéthoxyphosphorylméthoxy)-propoxyamino]-2,5-diformamidopyrimidine,
4-chloro-6-[[3-acétoxy-2(diéthoxyphosphorylméthoxy)-propoxy]-amino]-5-formamidopyrimidine et
4-chloro-6[[3-acétoxy-2-(diéthoxyphosphorylméthoxy)-propoxy]-amino]-2,5-diformamidopyrimidine.

**11.** Intermédiaire de formule (V) tel que défini dans la revendication 8, dans lequel Z est remplacé par un groupe aminooxy et dans lequel $R_4'$ est $R_4$ tel que défini dans la revendication 1.

**12.** Composé choisi parmi:
le (1-aminooxyméthyl-2-benzyloxyéthoxy)-méthylphosphonate de diéthyle et
le [2-acétoxy-1-(aminooxyméthyl)-éthoxy]-méthylphosphonate de diéthyle.

**13.** Utilisation d'un composé suivant l'une quelconque des revendications 1 à 7, dans la fabrication d'un médicament utile dans le traitement d'infections virales ou de maladies néoplastiques.

**14.** Utilisation de la 9-[2-(phosphonométhoxy)-éthoxy]-adénine dans la fabrication d'un médicament utile dans le traitement d'infection par le virus de l'immunodéficience humaine.